# EUROPEAN PATENT SPECIFICATION

(11) **EP 0 540 128 B1**
(45) Date of publication and mention of the grant of the patent: **07.08.1996**
(21) Application number: 92203892.2
(22) Date of filing: 26.09.1989
(51) Int. Cl.: C12N 15/12, A61K 39/00, C07K 14/435, C07K 16/18, A61K 39/395, G01N 33/569

(54) **Nematode vaccine**
Vakzin gegen Nematoden
Vaccin contre les nématodes

(30) Priority: 26.09.1988 AU 621/88
(43) Date of publication of application: 05.05.1993
(62) Divisional of application: 89910995.3
(73) Proprietor: BIOTECHNOLOGY AUSTRALIA PTY. LTD., Roseville, NSW 2069 (AU); COMMONWEALTH SCIENTIFIC AND INDUSTRIAL RESEARCH ORGANISATION, Campbell, Australian Capital Territory 2601 (AU)
(72) Inventor: Dopheide Theodorus Antonius Aloisius, Eltham 3095 Victoria (AU); Grant Warwick Norman, Armidale 2350 New South Wales (AU); Frenkel Maurice Joseph, South Caulfield 3162 Victoria (AU); Savin Keith William, South Caulfield 3162 Victoria (AU); Wagland Barry Maxwell, Carlingford 2118 New South Wales (AU)
(74) Representative: Bannerman, David Gardner

(56) References cited:
- INT. J. PARASITOL. vol. 15, no. 2, 1985, pages 129 - 136 I. O'DONNELL ET AL. 'Attempts to probe the antigens and protective immunogens of Trichostrongylus colubriformis'

## Description

### TECHNICAL FIELD

The invention relates to the identification of antigens which induce protective immunity in a host against infection by parasitic nematode species, such as species of the genera Trichinella, Ancyclostoma, Strongylus, Trichostrongylus, Haemonchus, Ostertagia, Ascaris, Toxascaris, Uncinaria, Trichuris, Dirofilaria, Toxocara, Necator, Enterobius, Strongyloides and Wuchereria, especially the genera Trichostrongylus and Haemonchus. Examples of such species include Trichinella spiralis, Ancylostoma caninum, Strongylus vulgaris, Trichostrongylus colubriformis, Haemonchus contortus, Ostertagia ostertagi, Ascaris suum, Toxascaris leonina, Uncinaria stenocephala, Trichuris vulpis, Dirofilaria immitis, the larvae of Toxocara spp., Necator americanus, Ancylostoma duodenale, Ascaris lumbricoides, Trichuris trichiura, Enterobius vermicularus, Strongyloides stercoralis and Wuchereria bancrofti, particularly Trichostrongylus colubriformis and Haemonchus contortus.

The invention also relates to nucleotide sequences encoding these antigens, as well as to recombinant DNA molecules containing such nucleotide sequences and host cells expressing these nucleotide sequences.

The invention further relates to methods for the production of the antigens, nucleotide sequences, recombinant DNA molecules and hosts of the invention.

The invention relates to antibodies raised against the antigens of the invention and to compounds which act in a manner similar to those antibodies.

Additionally, the invention relates to vaccines which induce protective immunity against infection by parasitic nematodes such as species of the genera Trichinella, Ancyclostoma, Strongylus, Trichostrongylus, Haemonchus, Ostertagia, Ascaris, Toxascaris, Uncinaria, Trichuris, Dirofilaria, Toxocara, Necator, Enterobius, Strongyloides, and Wuchereria, especially the genera Trichostrongylus and Haemonchus. Examples of such species include Trichinella spiralis or Ancylostoma caninum in man, Strongylus vulgaris in horses, Trichostrongylus colubriformis in sheep and goats, Haemonchus contortus in sheep and goats, Ostertagia ostertagi in cattle, Ascaris suum or Trichinella spiralis in pigs, Toxascaris leonina or Uncinaria stenocephala in cats, Ancylostoma caninum or Trichuris vulpis in dogs, Dirofilaria immitis in dogs, or the larvae of Toxocara spp in man, or infection by Necator americanus, Ancylostoma duodenale, Ascaris lumbricoides, Trichuris trichiura, Enterobius vermicularus, Strongyloides stercoralis or Wuchereria bancrofti, and particularly Trichostrongylus colubriformis or Haemonchus contortus.

### BACKGROUND ART

Nematodes (nema - thread; oides - resembling), which are unsegmented roundworms with elongated, fusiform, or saclike bodies covered with cuticle, are virtually ubiquitous in nature, inhabiting soil, water and plants, and are importantly involved in a wide range of animal and plant parasitic diseases.

The roundworm parasites of mammals belong to the phylum Nemathelminthes. The roundworms include the hookworm (e.g. Necator americanus and Ancylostoma duodenale), roundworm (e.g. the common roundworm Ascaris lumbricoides), whipworm (e.g. Trichuris trichiura), and the pinworm or threadworm (e.g. Enterobius vermicularus), as well as Strongyloides stercoralis, Trichinella spiralis and the filarial worm Wuchereria bancrofti. Other important roundworm parasites include Ancylostoma caninum (infections of man), Strongylus vulgaris (infections of horses), Trichostrongylus colubriformis, Ostertagia circumcincte (infections of sheep and goats), Haemonchus contortus (infections of sheep and goats), Ostertagia ostertagi, Haemonchus placei (infections of cattle), Ascaris suum (infections of pigs), Toxascaris leonina or Uncinaria stenocephala (infections of dogs), Toxocara spp (circulatory infections of man) and Dirofilaria immitis (circulatory infections of cats and dogs).

Even when symptom-free, parasitic worm infections are harmful to the host animal for a number of reasons; e.g. they deprive the host of food, injure organs or obstruct ducts, may elaborate substances toxic to the host, and provide a port of entry for other organisms. In other cases, the host may be a species raised for food and the parasite may be transmitted upon eating to infect the ingesting animal. It is highly desirable to eliminate such parasites as soon as they have been discovered.

More commonly, such infections are not symptom-free. Helminth infections of mammals, particularly by parasitic nematodes, are a source of great economic loss, especially of livestock and pets, e.g. sheep, cattle, horses, pigs, goats, dogs, cats, and birds, especially poultry (see CSIRO/BAE Report - "Socio-economic Developments and Trends in the Agricultural Sector: Implications for Future Research"). These animals must be regularly treated with anthelminthic chemicals in order to keep such infections under control, or else the disease may result in anaemia, diarrhoea, dehydration, loss of appetite, and even death.

The only currently available means for controlling helminth infections is with the use of anthelminthic chemicals, but these are only effective against resident worms present at the time of treatment. Therefore, treatment must be continuous since the animals are constantly exposed to infection; e.g. anthelminthic treatment with diethylcarbamazine is required every day or every other day most of the year to control Dirofilaria immitis or the dog heartworm. This is an expensive and labour intensive procedure. Due to the widespread use of anthelminthic chemicals, the worms may develop resistance and so new and more potent classes of chemicals must be developed. An alternative approach is clearly desirable.

The development of a vaccine against parasitic nematodes would overcome many of the drawbacks inherent in chemical treatment for the prevention and curing of helminthic infections. The protection would certainly last longer, only the vaccinated animal would be affected, and the problems of toxicity and persistence of residues would be minimized or avoided. Accordingly, there have been several reported attempts to develop such vaccines using parasitic nematodes; unfortunately, they have met with limited success and factors such as material availability and vaccine stability have precluded their large scale use.

One such attempt described by J.K. Dineen, (1977) involves the use of irradiated larval vaccines. As with other such attempts, the utility of this method is restricted by the requirement to maintain viable nematodes for prolonged periods.

The failure of killed vaccine preparations to afford good anthelminthic protection has been thought to be due to a number of factors. For example, it has been considered by J.T.M. Neilson (1975) that parasitic nematodes may have evolved mechanisms by which they can secrete products which immunosuppress or immunomodulate the host's immune system, thereby both preventing the development of an effective immune response and rendering the host susceptible to other infections. It is believed by Dineen and Wagland (1982), that immunosuppressants or immunomodulators may be present in the crude preparations of parasitic nematodes which are used in the killed vaccines. A second problem suggested by this review article is that parasitic nematodes may have altered their antigen profile to one which resembles that of the host so that, in a natural infection, vigorous immunlogical reactions are not provoked by protective parasitic antigens. Such a phenomenon would also occur following vaccination with impure preparations of killed nematodes or extracts thereof.

Some workers have shown accelerated explusion of worms from host animals using whole homogenates of worms and impure subfractions see for example Rothwell and co-workers (1974, 1977, 1979), O'Donnell et at (1985), Neilson and Van de Walle (1987), Silverman: U.S. Patent 894603, Australian Patent 247 354, Adams (1989), East et al (1989), Munn and Greenwood (1987) (Australian Patent Application No. 77590/87), Connan (1965), Savin et al (1988) and McGillivery et al (1988).

In all of these studies, crude extracts of nematodes have been used to vaccinate animals, and no defined antigen or individual components of the extracts have been identified as being responsible for protection.

There have been some reports attempting to identify purified protective components, see for example Silberstein and Despommier (1985), Hoetz et al (1985), Grandea et al (1989), Lucius et al (1988), Donelson et al (1988), Nilsen et al (1988). However, protection has either not been shown or not substantiated for the components described.

In only one natural host/parasitic nematode system has a purified cloned subunit been shown to be protective. In Australian Patent Application No. 19998/88, it was demonstrated that a recombinant DNA derived antigen shown to be nematode tropomyosin, gave 50% protection in sheep against Haemonchus contortus challenge. For reasons which will become clear later in this specification, this antigen is different to those identified in the current specification: the current antigens being found in the excretory/secretory fluids of nematodes following incubation in vitro.

The CSIRO/BAE working paper "Socio-economic Developments and Trends in the Agricultural Sector: Implications for Future Research" cited intestinal parasites as one of the three most urgent health problems in the Australian sheep industry and indicated that the development of vaccines holds great promise for better control of these infections.

It is well established that animals which are infected with parasitic nematodes develop an immunity which renders them less susceptible to subsequent infection (see Rothwell 1989 for review).

Although it has been demonstrated (e.g. O'Donnell et al 1985) that many parasite proteins are recognised by the immune system of infected host animals during parasitic infection, many of the immune responses will have no functional significance in terms of resistance to re-infection. The major step is to identify, from the many thousands of proteins present in the parasitic organism, the individual proteins which can induce immune responses in the host animal that protect it from re-infection.

Recent advances in biotechnology and in particular recombinant DNA technology, realistically offer the opportunity to produce commercially-viable vaccines against a range of economically-important parasites of man and domestic animals. This approach would overcome many of the problems proposed to account for the lack of efficacy of killed vaccines using crude parasite preparations. For example, the vaccines produced by recombinant DNA techniques would not contain immunosuppressants or immunomodulators which may be found in crude extracts of parasitic nematode species. But it is necessary to first identify the antigens. Once identified and characterised, recombinant DNA technology could be used to construct microorganisms which synthesize those proteins or portions of the proteins containing protective epitopes and use the products synthesized by the recombinant organism in vaccines to protect animals from infection with the parasites.

The present inventors have studied in detail the excretory/secretory products from adult T. colubriformis and components from the mixture which are capable of giving protection following vaccination of target animals have been purified and characterised at the molecular level.

### DESCRIPTION OF INVENTION

The present inventors have found that protective immunity against infection by parasitic nematodes can be induced by immunization with excretory/secretory products of a parasitic nematode species. Five molecules termed Tc Ad ESA1, Tc Ad ESA2, Tc Ad ESA3, Tc Ad ESA4 and Tc Ad ESA5 are described which have been purified from the excretory-secretory fluids of mature adults of T. colubriformis and characterized. The present inventors have found that on vaccination, these proteins induce protective responses in guinea pigs against infection with T. colubriformis.

Adult worms were recovered from sheep 21 days after infection, washed and maintained in RPMI 1640 culture medium, containing antibiotics at 37°C for 16 hours. This culture medium which contains the excretory/secretory fluids from T. colubriformis, was concentrated over Diaflo membranes, and fractionated by adsorption to a lentil lectin-Sepharose 4B column.

The unbound fraction (LL⁻) and the bound fraction (LL⁺, eluted with methylmannoside) each contained only a few protein bands and were fractionated further by polyacrylamide gel electrophoresis and electroelution. Three proteins, designated Tc Ad ESA1, Tc Ad ESA2 and Tc Ad ESA5 have been isolated from the lentil lectin bound fraction and a further two proteins designated Tc Ad ES3 and Tc Ad ES4 were isolated from the unbound fraction. All five proteins confer immunity to T. colubriformis infection following intraperitoneal injection of guinea pigs, a laboratory model for sheep.

Examples of the antigens of the invention are the purified proteins Tc Ad ESA1, Tc Ad ESA2, Tc Ad ESA3, Tc Ad ESA4 and Tc Ad ESA5 having molecular weights of 30, 37, 17, 11 and 81kD respectively as estimated by SDS-PAGE.

According to a first embodiment of this invention there is provided an antigen comprising: an excretory/secretory protein derived from a first parasitic nematode species and capable of inducing protective immunity against infection of a host by a second parasitic nematode species, which may be the same as or different from the first nematode species; or a protein molecule comprising all, part, an analogue, homologue, derivative or combination thereof of the excretory/secretory protein, which protein molecule is capable of inducing protective immunity in a host against infection by a parasitic nematode.

Preferably, the excretory/secretory protein has an approximate molecular weight of 11, 17, 30, 37 or 81 kD as estimated by SDS-PAGE.

Typically, the first parasitic nematode species is selected from species of the genera Trichinella, Ancylostoma, Strongylus, Trichostrongylus, Haemonchus, Ostertagia, Ascaris, Toxascaris, Uncinaria, Trichuris, Dirofilaria, Toxocara, Necator, Enterobius, Strongyloides and Wuchereria. Examples of such species include Trichinella spiralis, Ancylostoma caninum, Strongylus vulgaris, Trichostrongylus colubriformis, Haemonchus contortus, Ostertagia ostertagi, Ascaris suum, Toxascaris leonina, Uncinaria stenocephala, Trichuris vulpis, Dirofilaria immitis, Toxocara spp, Necator americanus, Ancylostoma duodenale, Ascaris lumbricoides, Trichuris trichiura, Enterobius vermicularus, Strongyloides stercoralis and Wuchereria bancrofti.

Typically, the second parasitic nematode species is selected from species of the genera Trichinella, Ancylostoma, Strongylus, Trichostrongylus, Haemonchus, Ostertagia, Ascaris, Toxascaris, Uncinaria, Trichuris, Dirofilaria, Toxocara, Necator, Enterobius, Strongyloides and Wuchereria. Examples of such species include Trichinella spiralis, Ancylostoma caninum, Strongylus vulgaris, Trichostrongylus colubriformis, Haemonchus contortus, Ostertagia ostertagi, Ascaris suum, Toxascaris leonina, Uncinaria stenocephala, Trichuris vulpis, Dirofilaria immitis, Toxocara spp, Necator americanus, Ancylostoma duodenale, Ascaris lumbricoides, Trichuris trichiura, Enterobius vermicularus, Strongyloides stercoralis and Wuchereria bancrofti.

Preferably, the first parasitic nematode species is T. colubriformis.

Preferably, the second parasitic nematode species is T .colubriformis or H. contortus.

According to a second embodiment of this invention there is provided: a first nucleotide sequence encoding the amino acid sequence of an antigen of the first embodiment; a nucleotide sequence which hybridizes to the first nucleotide sequence; or a nucleotide related by mutation including single or multiple base substitutions, insertions or deletions to the first nucleotide sequence.

Preferred nucleotide sequences of the invention are those encoding the excretory/secretory proteins of the first embodiment having approximate molecular weights of 11, 17, 30, 37 and 81kD as estimated by SDS-PAGE.

Preferably, the nucleotide sequence is a DNA sequence. The DNA sequences embraced by the present invention can be prepared, for example, from T. colubriformis cells by extracting total DNA therefrom and isolating the sequences by standard techniques. Alternatively, the DNA may be prepared in vitro, synthetically or biosynthetically, such as by the use of an mRNA template.

According to a third embodiment of this invention there is provided a process for selecting a DNA or RNA sequence coding for an antigen according to the first embodiment which process comprises providing one or more DNA or RNA sequences and determining which of the sequences hybridizes with a DNA or RNA sequence known to code for an antigen of the first embodiment or providing an antiserum to the antigen and identifying host-vector combinations that express the antigen.

The sequences may be from natural sources, may be RNA sequences, synthetic sequences, DNA sequences from recombinant DNA molecules or combinations of such sequences.

Preferably, the process used to identify and characterize DNA coding for the antigen involves the extraction of mRNA species from cells producing the antigen, their conversion to double stranded DNA (cDNA) and the insertion of these into an autonomously replicating factor, such as a plasmid or phage vector. This is followed by transformation of a host cell such as a bacterial strain with the factor and screening of the library produced with synthetic DNA probes which are complementary to the antigen encoding mRNA or DNA sequences in order to detect those clones which contain DNA coding for the antigen as opposed to any other cell proteinaceous components.

According to a fourth embodiment of this invention, there is provided a recombinant DNA molecule comprising a DNA sequence of the third embodiment and vector DNA.

The DNA sequence may be a natural, synthetic or biosynthetic DNA sequence.

Preferred recombinant DNA molecules of the invention include an expression control sequence operatively linked to the DNA sequence.

In one preferred form of the invention, the DNA sequence is operatively linked to the β-galactosidase gene of E. coli. Other preferred control systems include those of the tryptophan (Trp) operon, the Tra-T gene of E. coli, the leftward promoter of bacteriophage lambda, the Cup 1 promoter and hybrid promoters such as tac or viral promoters such as the SV40 early promoter.

Preferably, the vector DNA is plasmid DNA. Suitable plasmid vectors include pUR290, pUC18, pYEUC114 and derivatives thereof.

Alternatively, the vector DNA may be bacteriophage DNA such as bacteriophage lambda and derivatives thereof, such as lambda gt11 and lambda gt10.

According to a fifth embodiment of this invention there is provided a fused gene comprising a promoter, a translation start signal and a DNA sequence of the third embodiment.

According to a sixth embodiment of this invention there is provided a process for the preparation of a recombinant DNA molecule of the fourth embodiment which process comprises providing a DNA insert comprising a DNA sequence of the third embodiment and introducing the DNA insert into a cloning vector.

Preferably, the DNA insert is introduced into the cloning vector in correct spacing and correct reading frame with respect to an expression control sequence.

According to a seventh embodiment of this invention there is provided a host transformed with at least one recombinant DNA molecule of the fourth embodiment.

Preferably, the transformed host is capable of expressing an antigen of the first embodiment.

Suitable hosts include bacterial cells, yeasts such as Saccharomyces cerevisiae strain CL13-ABSY86 , other fungi, vertebrate cells, insects cells, plant cells, human cells, human tissue cells live viruses such as vaccinia and baculovirus and whole eukaryotic organisms.

Suitable bacterial hosts include E. coli and other enteric organisms, Pseudomonas, and Bacillus species.

Preferred hosts are E. coli K12 derivatives; in particular JM109 and Y1090.

According to an eighth embodiment of this invention there is provided a process for transforming a host to provide a transformed host of the seventh embodiment which process comprises providing a host, making the host competent for transformation, and introducing into the host a recombinant DNA molecule of the fourth embodiment.

According to a ninth embodiment of this invention there is provided an expression product of a transformed host of the seventh embodiment which product comprises an antigen of the first embodiment.

Preferably, the expression product is provided in substantially pure form.

Preferably, the expression product comprises a first polypeptide sequence homologous to the host and a second polypeptide sequence which is an amino acid sequence coding for an antigen of the first embodiment.

More preferably, the first amino acid sequence is part or all of β-galactosidase or Tra-T and the host cell is E. coli.

According to a tenth embodiment of this invention there is provided a process for the biosynthesis of a proteinaceous product comprising an antigen of the first embodiment which process comprises:
transforming a host with a recombinant DNA molecule of the fourth embodiment so that the host is capable of expressing a proteinaceous product which includes an antigen of the first embodiment; culturing the host to obtain expression; and collecting the proteinaceous product.

According to an eleventh embodiment of this invention there is provided an epitope of an antigen of the first embodiment which is responsible for the protective immune response. The epitope may be created artificially by the synthetic production of oligopeptides which contain sequences of portions of the antigen which can be predicted from the results of immunochemical tests on fragments of the proteins produced in bacteria or generated as a result of chemical or enzymatic cleavage of the native or recombinant peptides.

According to a twelfth embodiment of this invention there is provided an antibody generated against an epitope of the eleventh embodiment. These antibodies or idiotypes can be used for passive protection of animals.

According to a thirteenth embodiment of this invention there is provided an antibody generated against the variable region of an antibody of the twelfth embodiment, a so called anti-idiotype antibody, which mimics a protective epitope of the antigen and may be used as an effective vaccine in active immunization of animals.

According to a fourteenth embodiment of this invention there is provided a vaccine comprising an effective amount of one or more antigens of the first embodiment, expression products of the ninth embodiment, epitopes of the eleventh embodiment and/or anti-idiotype antibodies of the thirteenth embodiment, together with a pharmaceutically acceptable excipient, carrier, adjuvant and/or diluent.

Preferred vaccines include those suitable for injectable or oral administration. Preferably, injectable vaccines include a pharmaceutically acceptable adjuvant.

According to a fifteenth embodiment of this invention there is provided an antibody prepared as a result of vaccination of a host by administration of one or more antigens, expression products, epitopes, anti-idiotype antibodies and/or vaccines of the present invention to the host. Such antibodies include polyclonal and monoclonal antibodies.

It is recognised that there are compounds which act in a manner similar to the antibodies of the fifteenth embodiment. Although these compounds are not antibodies their presence in the host can produce a similar protective effect to the antibodies. Throughout the specification and claims, reference to antibodies of the fifteenth embodiment should be construed as extending to these compounds.

According to a sixteenth embodiment of this invention there is provided: an antibody composition comprising at least one antibody of the twelfth and/or fifteenth embodiment together with a pharmaceutically acceptable carrier, diluent and/or excipient.

According to a seventeeth embodiment of this invention, there is provided a process for the preparation of an antigen of the first embodiment which process comprises: collecting excretory-secretory fluids from a parasitic nematode species; fractionating the fluid by lentil lectin chromatography with methylmannoside as eluent; collecting the bound and unbound fractions; further fractionating by SDS-gel electrophoresis; and electroeluting the antigen.

According to an eighteenth embodiment of this invention there is provided a process for the preparation of a fused gene of the fifth embodiment which process comprises providing a promoter, a translation start signal and a DNA sequence of the third embodiment and operatively linking the promoter, translation start signal and DNA sequence.

According to a nineteenth embodiment of this invention there is provided a process for the preparation of a vaccine of the fourteenth embodiment which process comprises admixing an effective amount of at least one antigen of the first embodiment and/or expression product of the ninth embodiment and/or epitope of the eleventh embodiment and/or anti-idiotype antibody of the thirteenth embodiment with a pharmaceutically acceptable carrier, diluent, excipient and/or adjuvant.

According to a twentieth embodiment of this invention there is provided a process for the preparation of an antibody of the fifteenth embodiment which process comprises immunizing an immunoresponsive host with an antigen of the first embodiment and/or expression product of the ninth embodiment and/or epitope of the eleventh embodiment and/or anti-idiotype antibody of the thirteenth embodiment and/or a vaccine of the fourteenth embodiment.

According to a twenty-first embodiment of this invention there is provided a process for the preparation of an anti-idiotype antibody of the thirteenth embodiment which process comprises immunizing an immunoresponsive host with an antibody of the twelfth embodiment.

According to a twenty-second embodiment of this invention there is provided a process for the preparation of an antibody composition of the sixteenth embodiment which process compries: admixing an effective amount of at least one antibody of the twelfth and/or fifteenth embodiment with a pharmaceutically acceptable carrier, diluent and/or excipient.

According to a twenty-third embodiment of this invention there is provided a method of protecting a host in need of such treatment from infection by a parasitic nematode species which method comprises vaccinating the host with an antigen, expression product, vaccine, epitope and/or anti-idiotype antibody of the invention.

According to a twenty-fourth embodiment of this invention there is provided a method of passively protecting a host in need of such treatment against infection by a parasitic nematode species which method comprises passively vaccinating the host with at least one antibody of the twelfth and/or fifteenth embodiment and/or antibody composition of the sixteenth embodiment.

The amount of antigen, expression product, epitope and/or anti-idiotype antibody that may be combined with carrier, excipient, diluent and/or adjuvant to produce a single vaccine dosage form will vary depending upon the infection being treated or prevented, the host to be treated and the particular mode of administration.

It will be understood, also, that the specific dose level for any particular host will depend upon a variety of factors including the activity of the specific antigen, expression product, epitope, anti-idiotype antibody and/or vaccine employed, the age, body weight, general health, sex, diet, time of administration, route of administration, rate of excretion, drug combination, the particular infection to be treated or prevented and the severity of the particular infection undergoing treatment or prevention.

The vaccine of the present invention may be administered orally or parenterally, in unit dosage formulations containing conventional, non-toxic, pharmaceutically acceptable carriers, diluents, adjuvants and/or excipients as desired.

Injectable preparations, for example, sterile injectable aqueous or oleagenous suspensions may be formulated according to known arts using suitable dispersing or wetting agents and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The term "pharmaceutically acceptable adjuvant" can mean either the standard compositions which are suitable for human administration or the typical adjuvants employed in animal vaccinations. An appropriate adjuvant can be selected using ordinary skill in the art.

Suitable adjuvants for the vaccination of animals and humans include but are not limited to aluminium hydroxide and oil emulsions such as Marcol 52: Montanide 888 (Marcol is a Trademark of Esso. Montanide is a Trademark of SEPPIC, Paris.). Other adjuvants suitable for use in the present invention include conjugates comprising the expression product together with an integral membrane protein of prokaryotic or eukaryotic origin, such as TraT.

Routes of administration, dosages to be administered as well as frequency of injections are all factors which can be optimized using ordinary skill in the art. Typically, the initial vaccination is followed some weeks later by one or more "booster" vaccinations, the net effect of which is the production of vigorous immunological responses such as high titres of antibodies against the antigen epitope, anti-idiotype antibody or expression product.

Solid dosage forms for oral administration may include capsules, tablets, pills, powders, and granules. In such solid dosage forms, antigens, epitopes, anti-idiotype antibodies and/or expression products may be admixed with at least one inert diluent such as sucrose, lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include nanoparticles, microcapsules, LTB conjugates, cholera or its B subunit as a conjugate, in pharmaceutically acceptable emulsions, syrups, solutions, suspensions, and elixirs containing inert diluents commonly used in the art, such as water. Such compositions may also comprise adjuvants, such as wetting agents, emulsifying and suspending agents or TraT as a conjugate, and sweetening, flavouring, and perfuming agents including sugars such as sucrose, sorbitol, fructose, etc., glycols such as polyethylene glycol, propylene glycol etc, oils such as sesame oil, olive oil, soybean oil etc., antiseptics such as alkylparaphydroxybenzoate etc, and flavours such as strawberry flavour, peppermint etc.

### BRIEF DESCRIPTION OF THE DRAWINGS

Figure 1 illustrates SDS-PAGE analysis of the LL⁺ and LL⁻ fractions.

Figure 2 illustrates SDS-PAGE analysis of Tc Ad ESA 1,2,3,4 and 5.

Figure 3 illustrates SDS-PAGE analysis of Tc Ad ESA1 before and after deglycosylation.

Figure 4 illustrates the structure of a Tra T-Tc Ad ESA1 fusion.

Figure 5 illustrates the yeast expression vector pYEUC114 used to express Tc Ad ESA1 in Saccharomyces cerevisiae.

Figure 6 illustrates the detection of ESA encoding sequences in H. contortus and Dirofilaria immitis.

### BEST MODE AND OTHER MODES OF CARRYING OUT THE INVENTION

The nucleotide sequences, fused genes, recombinant DNA molecules and transformed hosts of the invention are prepared using standard techniques of molecular biology such as those described in Maniatis et al (1982).

In preparing the nucleotide sequences of the invention, it is recognised that the genes of interest, and also cDNA copies made from the genes may be provided in low yield. PCR (polymerase chain reaction) techniques can be used to amplify the relevant DNA to faciliate detection and cloning.

Expression products of the invention are obtained by culturing the transformed hosts of the invention under standard conditions as appropriate to the particular host and separating the expression product from the culture by standard techniques. The expression product may be used in impure form or may be purified by standard techniques as appropriate to the expression product being produced and the particular host.

The vaccines of the invention are prepared by mixing, preferably homogeneously mixing, antigen, expression product, anti-idiotype antibody and/or epitope with a pharmaceutically acceptable carrier, diluent, excipient and/or adjuvant using standard methods of pharmaceutical preparation.

The amount of antigen, expression product, anti-idiotype antibody and/or epitope required to produce a single dosage form will vary depending upon the infection to be treated or prevented, the host to be treated and the particular mode of administration. The specific dose level for any particular host will depend upon a variety of factors including the activity of the antigen, expression product, anti-idiotype antibody and/or epitope employed, the age, body weight, general health, sex, and diet of the host, time of administration, route of adminstration, rate of excretion, drug combination and the severity of the infection undergoing treatment.

The vaccine may be administered orally or parenterally in unit dosage formulations containing conventional, non-toxic, pharmaceutically acceptable carriers, diluents, excipients and/or adjuvants as desired.

Antibodies are raised using standard vaccination regimes in appropriate hosts. The host is vaccinated with an antigen, expression product, epitope, anti-idiotype antibody and/or vaccine of the invention.

The compounds acting in a similar manner to the antibodies of the invention may be purified naturally occuring compounds or synthetically prepared using standard techniques including standard chemical or biosynthetic techniques.

The antibody composition is prepared by mixing, preferably homogeneously mixing, antibody with a pharmaceutically acceptable carrier, diluent and/or excipient using standard methods of pharmaceutical preparation.

The amount of antibody required to produce a single dosage form will vary depending upon the infection to be treated or prevented, host to be treated and the particular mode of administration. The specific dose level for any particular host will depend upon a variety of factors including the activity of the antibody employed, the age, body weight, general health, sex and diet of the host, time of administration, route of administration, rate of excretion, drug combination and the severity of the infection undergoing treatment.

The antibody composition may be administered orally or parenterally in unit dosage formulations containing conventional, non-toxic, pharmaceutically acceptable carriers, diluents and/or excipients as desired.

The invention is further described with reference to the following Examples.

### Example 1

### Preparation of excretory/secretory antigens (ESA) from adult T. colubriformis.

Young merino Border Leicester cross bred lambs 12 months old and reared worm free were infected with 60,000 infective larvae of T. colubriformis. Twenty one days post-infection, the sheep were slaughtered and the nematodes were recovered from the intestine by Baermanization. The worms were washed in RPMI 1640 culture medium containing penicillin (100 units/ml) and streptomycin (100µg/ml) and incubated in the same medium (approximately 1000 worms/ml) for 16h at 37°C in an incubator with 5% CO₂. The viability of the worms was monitored by visual inspection and routinely more than 95% were alive and motile.

The worms and large debris were removed from the culture media by filtration or centrifugation. The supernatant or filtrate thus obtained is referred to as adult ESA (Tc Ad ESA).

Similar preparations referred to as Tc L4 ESA have been made from T. colubriformis fourth stage larvae recovered from sheep after 7-8 days infection. The subsequent analysis of the components of the extracts by polyacrylamide gel electrophoresis in the presence of sodium dodecylsulphate (SDS-PAGE) showed that L4 and adult extracts contained similar antigens but the extracts from the adults have been used in preference as they yielded more material than L4 extracts.

### Example 2

### Vaccination of guinea pigs with L4 ESA and Adult ESA.

Excretory/secretory antigens were prepared from L4 and adult T. colubriformis as described in Example 1. This material was used to vaccinate guinea pigs intraperitoneally using the procedure described by O'Donnell et al (1985). It can be seen (Tables 1 and 2) that the ESA from L4 or young adult nematodes gave highly significant protection in each experiment (62-92% reduction in parasitism).

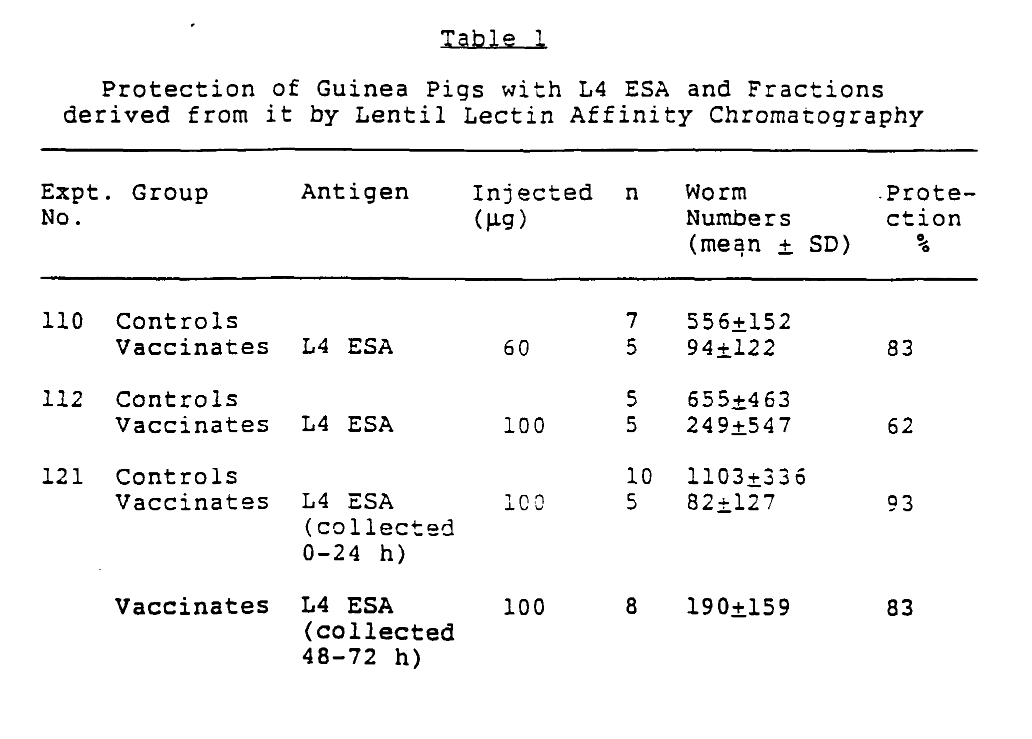

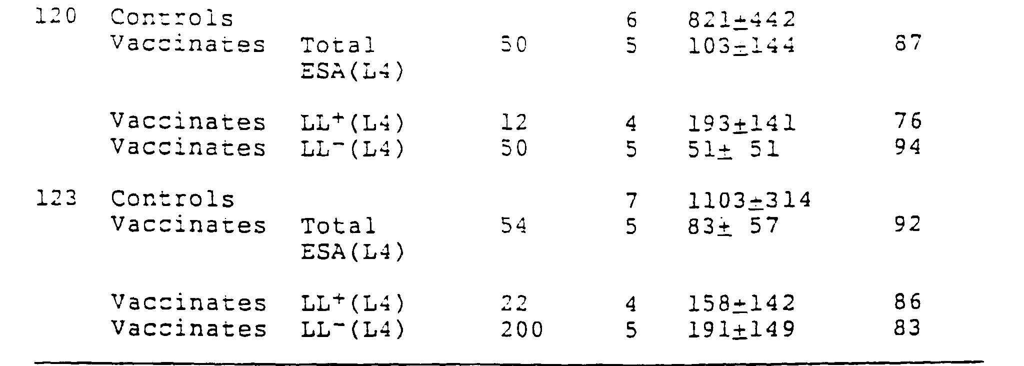

Vaccinates were injected intraperitoneally with the relevant antigen (n indicates the number of guinea pigs in each group). Animals were challenged with 2000 larvae 28 days later and killed for worm counts 13 days post challenge. LL⁺ is material bound and eluted from the lentil lectin column. LL⁻ is the unbound, run through material.

**Table 2**

| Protection of Guinea Pigs with Adult ESA and Fractions Derived from it by Lentil Lectin Affinity Chromatography | | | | | | |
|---|---|---|---|---|---|---|
| Expt. No. | Group | Antigen | Injected (µg) | n | Worm Numbers | Protection % |
| 126 | Controls | | | 8 | 1069±343 | |
| | Vaccinates | Ad ESA | 100 | 8 | 300±239 | 72 |
| | | | | | | |
| 164 | Controls | | | 5 | 910±243 | |
| | Vaccinates | Ad ESA | 50 | 5 | 140±211 | 85 |
| | Vaccinates | Ad ESA LL⁺ | 25 | 4 | 219±277 | 76 |
| | Vaccinates | Ad ESA LL⁺ | 50 | 4 | 544±348 | 40 |
| | | | | | | |
| 280 | Controls | | | 11 | 1484±375 | |
| | Vaccinates | Ad ESA | 10 | 9 | 252±435 | 83 |

Vaccinates were injected intraperitoneally with the relevant antigen (n indicates the number of guinea pigs in each group). Animals were challenged with 2000 larvae 28 days later and killed for worm counts 13 days post challenge. The material bound by the lentil lectin column is LL⁺; that unbound is LL⁻.

### Example 3

### Fractionation of adult ESA

The culture supernatant was concentrated 40 fold on a "Diaflo" (Amicon) YM10 membrane. The concentrated fluid was absorbed onto a lentil lectin Sepharose-4B (Pharmacia) column (5 x 1cm) equilibrated with Tris-buffered saline (TBS; 10mM Tris, 150mM NaCl, pH7.4). The column was washed with 100ml of TBS at a flow rate of 1ml/min and fractions containing unabsorbed material (measured by absorbance at 280nm) were collected. The specifically bound glycopeptides were eluted from the column using a solution of 2% methylmannoside in TBS. Fractions containing material absorbing at 280nm were pooled. Both the lentil lectin bound (LL⁺) and unbound (LL⁻) components were recipitated from solution by the addition of 10 volumes of methanol, chilling the mixture at -20°C for 16 hours and centrifugation at 12,000 xg for 15 min.

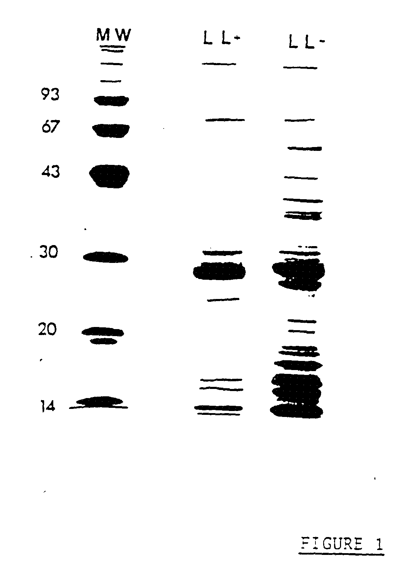

When analysed by SDS-PAGE (Fig 1) the LL⁺ fraction contained Coomassie staining bands with apparent molecular weights 81, 37, 32 and 30 kilodaltons (kD) together with some smaller molecular weight material when compared with molecular weight standards. The LL⁻ fraction contained several components including predominant bands at about 28-32, 17 and 10-12 kilodaltons.

### Example 4.

### Vaccination of guinea pigs with Lentil Lectin fractionated L4 and adult ESA.

The material prepared from L4 or adult ESA by lentil lectin chromatography was used to vaccinate guinea pigs intraperitoneally (O'Donnell et al 1985). Both the bound material and the unbound fraction gave highly significant degrees of protection to subsequent challenge of those guinea pigs with T. colubriformis (Tables 1 and 2). It is thus clear that there are components in both the bound and flow through fractions which are capable of eliciting a protective immune response following vaccination.

### Example 5.

### Further fractionation of lentil lectin bound and unbound components of adult ESA and recovery of individual antigens from preparative SDS gels.

Samples of the LL⁺ and LL⁻ fractions from adult ESA (100-500mg protein) were suspended in Laemmli buffer (Laemmli, 1970) and subjected to electrophoretic separation on preparative 12.5% SDS-polyacrylamide gels. Proteins were visualised with Coomassie R-250 and electroeluted (Stearne et al, 1985).

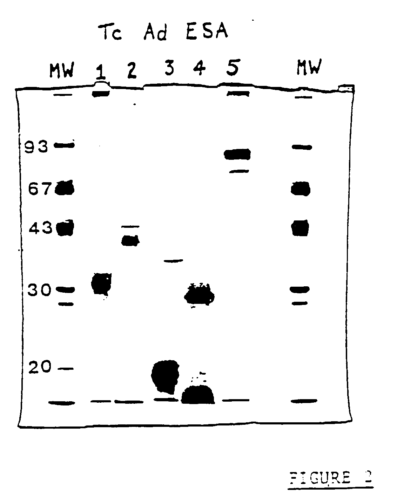

The components described here that were recovered from the LL⁺ fraction were Tc Ad ESA1, with an apparent molecular weight of 30kD; Tc Ad ESA2 with an apparent molecular weight of 37kD and Tc Ad ESA5 with an apparent molecular weight of 81kD (Fig.2). The components described here that were recovered from the LL⁻ fraction were Tc Ad ESA3 with an apparent molecular weight of 17kD and Tc Ad ESA4 with an apparent molecular weight of 11kD (Fig.2).

The LL⁺ 32kD component and the LL⁻ 28-30kD components (Fig. 1) are believed to be related to the LL⁺ 30kD antigen (Tc Ad ESA1), as western transfers resolved with antibodies raised against the purified Tc Ad ESA1 show cross-reaction with these components. These differences are likely to be due at least in part to differential degrees of glycosylation of the Tc Ad ESA1 as analysis of the cloned DNA sequence predicts that this component is extensively glycosylated.

### Example 6

### Vaccination of guinea pigs with purified antigens

The individual antigens electroeluted from SDS gels were used to vaccinate guinea pigs as described in Example 2. The guinea pigs were challenged with T. colubriformis and shown to be significantly protected from parasitism (Tables 3 and 4).

**Table 3**

| Protection of Guinea Pigs by Vaccination with Purified Antigens, Tc Ad ESA1, Tc Ad ESA2 and Tc Ad ESA5, from the lentil lectin bound fraction (LL⁺). | | | | | |
|---|---|---|---|---|---|
| Group | Antigen | Injected (µg) | n | Worm Numbers | Protection % |
| Experiment 200 | | | | | |
| Controls | | | 12 | 1135±263 | |
| Vaccinates 1 | Total LL⁺ | 60 | 5 | 354±341 | 69 |
| Vaccinates 2 | Tc Ad ESA1 | 21 | 5 | 458±534 | 60 |
| Vaccinates 3 | Tc Ad ESA2 | 24 | 5 | 482±683 | 57 |
| Vaccinates 4 | Tc Ad ESA5 | 8.5 | 5 | 530±361 | 53 |

| Experiment 218 | | | | | |
|---|---|---|---|---|---|
| Controls | | | 8 | 1389±773 | |
| Vaccinates | Total LL⁺ | 10 | 3 | 149±226 | 89 |
| Vaccinates | Tc Ad ESA1 | 20 | 4 | 563±828 | 59 |
| Vaccinates | Tc Ad ESA2 | 10 | 3 | 999±339 | 28 |
| Vaccinates | Tc Ad ESA5 | 10 | 3 | 706±283 | 49 |

| Experiment 257 | | | | | |
|---|---|---|---|---|---|
| Controls | | | 10 | 482±200 | |
| Vaccinates | Tc Ad ESA1 (deglycosylated) | 12.5 | 5 | 254±170 | 47 |

| Experiment 236 | | | | | |
|---|---|---|---|---|---|
| Controls | | | 11 | 1223±236 | |
| Vaccinates | Tc Ad ESA1 (deglycosylated) | 17 | 5 | 121±126 | 90 |

| Experiment 267 | | | | | |
|---|---|---|---|---|---|
| Controls | | | 7 | 652±281 | |
| Vaccinates | Tc Ad ESA1 (in Alhydrogel) | 10 | 5 | 238±178 | 63 |

Vaccinates were injected intraperitioneally with the relevant antigen. Animals were challenged with 2000 larvae 28 days later and killed for worm counts 13 days post challenge (n indicates the number of animals in each group).

**Table 4**

| Protection of Guinea Pigs by Vaccination with Purified Antigens Tc Ad ESA3 and Tc Ad ESA4 from the lentil lectinunbound fraction (LL⁻). | | | | | |
|---|---|---|---|---|---|
| Group | Antigen | Injected (µg) | n | Worm Numbers | Protection % |
| Experiment 236 | | | | | |
| Controls | | | 5 | 1103±186 | |
| Vaccinates 1 | Tc Ad ESA3 | 40 | 5 | 182±230 | 83 |
| Vaccinates 2 | Tc Ad ESA4 | 20 | 5 | 266±248 | 76 |

| Experiment 241 | | | | | |
|---|---|---|---|---|---|
| Controls | | | 10 | 773±609 | |
| Vaccinates 1 | Tc Ad ESA3 | 20 | 5 | 310±418 | 60 |
| Vaccinates 2 | Tc Ad ESA4 | 20 | 5 | 338±487 | 56 |

Vaccinates were injected intraperitoneally with antigens isolated from the adult T. colubriformis ESA preparations. Animals were challenged with 2000 infective larvae 28 days later and killed for worm counts 13 days post challenge.

It is clear from the results in Tables 3 and 4 that antigens electroeluted from SDS-PAGE of both the LL⁺ and LL⁻ fractions were capable of conferring substantial protection to guinea pigs against challenge infection by T. colubriformis. Of particular relevance in this work are the Tc Ad ESA1, Tc Ad ESA2 and Tc Ad ESA5 components of the LL⁺ fraction and the Tc Ad ESA3 and Tc Ad ESA4 components of the LL⁻ fraction. Other Tc Ad ESA components also had effects and are of relevance.

Vaccination of guinea pigs with Tc Ad ESA1 adjuvanted in Alhydrogel resulted in 63% protection being obtained. Deglycosylation of To Ad ESA1 did not result in a decrease in the extent of protection obtained (in experiment 257, the worm numbers in the controls were abnormally low) indicating that the protein portion of the molecule was capable of giving protection: the carbohydrate was apparently not the protective component.

### Example 7

### Amino acid sequence analysis of isolated peptides

The polypeptides isolated as described in Example 5 were analysed for N-terminal amino acid sequence on an Applied Biosystems gas phase amino acid sequencer. To obtain internal sequences, purified protein was digested with proteinase [37°C, overnight, in 0.1M NH₄HCO₃ pH 7.8 at 5% w/w enzyme/substrate ratio]. Peptides were separated by HPLC using a 30 x 2.1 mm Aquapore RP-300 column with a gradient of 0.1% TFA to 0.1% TFA/70% acetonitrile. Some of the amino acid sequences obtained are shown in Table 5: the underlined sequences were found to be particularly useful in providing information to design oligonucleotide probes suitable for isolation of cDNA clones.

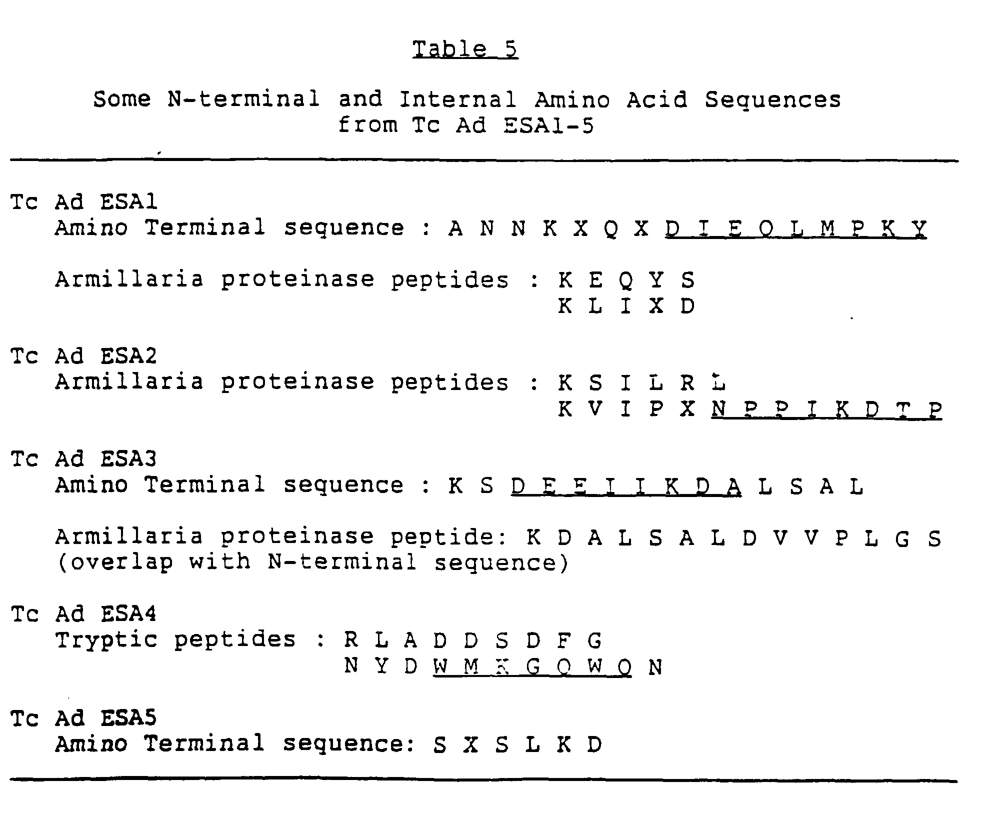

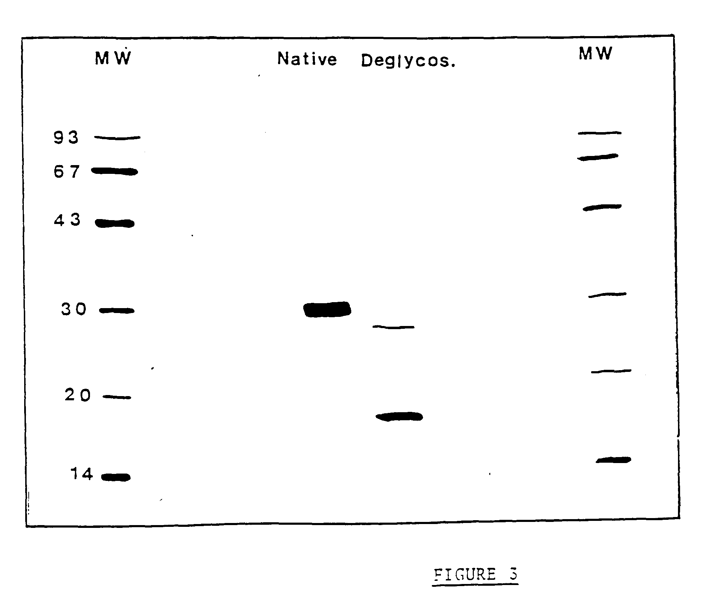

For Tc Ad ESA1, amino acid analysis after reduction and carboxymethylation (O'Donnell et al., 1973) indicated the presence of 2 residues of half-cystine. Deglycosylation of Tc Ad ESA1 with N-glycanase (Genzyme), which removes asparagine-linked carbohydrate, reduced the apparent molecular weight from 30kD to 15kD (Fig. 3). This is in close accordance with information provided by the cDNA sequence (see below).

Deglycosylation of Tc Ad ESA2 by the same treatment reduced the apparent molecular weight as analysed by SDS-PAGE from 37kD to approximately 30kD.

### Example 8.

### Isolation of recombinant organisms containing the genes coding for the Tc Ad ESA components

### A. Construction of cDNA Libraries.

Messenger RNA was isolated from the L4 stage of T. colubriformis by grinding the larvae in a buffer containing guanidine hydrochloride (6M) sodium acetate (0.2M pH 5.2), and 2-mercaptoethanol (50mM), precipitation with ethanol and fractionation on an oligo(dT)-cellulose column. The L4 PolyA⁺ mRNA was used as the template for synthesis of double-stranded cDNA using the Amersham ribonuclease H/DNA polymerase I kit (Amersham cDNA synthesis system, #RPN.1256) as recommended by the manufacturers. Following the addition of EcoRI linkers, the double-stranded cDNA was ligated to lambda gt11 and packaged into viable bacteriophage which were used to infect E. coli Y1090 cells, essentially as described by Huynh et al (1985). Using the above methods, a cDNA library was established consisting of 2 x 10⁵ independent recombinants. A similar technique was used to establish an adult cDNA library in lambda gt 10 containing 1.5 x 10⁵ independent recombinants.

### B. Oligonucleotide probes

i) Tc Ad ESA1
   The amino acid sequence D I E Q L M P was used to design a degenerate oligonucleotide probe 
5' G G C A T A A G T T G T T C A A T A T C 3'
             G     C     C     G     G
             C
ii) Tc Ad ESA2
   The amino acid sequence N P P I K D T P was used to design a degenerate oligonucleotide probe using deoxyinosine in positions of 4-fold degeneracy 
             A     T                     A
5' G G I G T G T C C T T I A T I G G I G G G T T 3'
iii) Tc Ad ESA3
   The amino acid sequence D E E I I K D A was used to design a degenerate oligonucleotide probe 
          A        T                          T        T        A
5' G C G T C C T T T A T T A T C T C C T C G T C
iv) Tc Ad ESA4
   The amino acid sequence W M K G Q W Q N was used to design an oligonucleotide probe 5' T T T T G C C A T T G I C C T T T C A T C C A 3'
v) Tc Ad ESA5

   G     T         G
5' A T C C T T I A A I G A I I I I G A 3'

All oligonucleotides are the reverse complement of the DNA sequence coding for the amino acid sequences selected.

### C. Selection of Recombinants from cDNA Libraries

The L4 and young adult cDNA libraries in lambda gt11 and gt10 respectively were amplified and aliquots were screened using the above synthetic oligonucleotides to probe duplicate filter lifts as described by Wallace et. al. [1985] and Benton and Davis [1977].

### D. Sequence of cDNA clones

A number of the selected clones contained an insert which could be resected with EcoRI and subcloned into M13mp18 digested with the same enzyme. The DNA sequence of the subcloned inserts were determined using the method of Sanger et. al. [1980]

The DNA sequence of several clones of the Tc Ad ESA1 cDNA was determined and is summarised in Table 6. The DNA sequence contains an open reading frame which codes for a protein of 130 amino acids. The N-terminal amino acid sequence corresponds to the sequence obtained by gas phase sequence analysis of the antigen isolated from Ad ESA1 (underlined in Table 6) and the two internal peptide sequences obtained from Armillaria mellea digests of Tc Ad ESA1 can also be identified. An E. coli stain TG1 transformed with plasmid vector pTTQ18 containing the Tc Ad ESA1 gene has been given the inhouse reference number BTA 1689.

Sequence of the DNA from several isolates has shown some variation in the translated amino acid sequence. The amino acids which have varied are doubly underlined in Table 6. The sequence corresponding to the mature protein has been determined. The sequence of the presumed N-terminal leader sequence has yet to be established.

The amino acid sequence shows four sites of potential N-linked glycosylation (consensus sequence AsnXSer/Thr) which is consistent with the lentil lectin binding properties of this antigen and with the altered mobility of the antigen in SDS-PAGE following treatment with N-glycanase. Finally, the molecular weight calculated from the amino acid sequence shown (15,300 daltons) is in close agreement with that obtained for the N-glycanase treated antigen (Fig. 3).

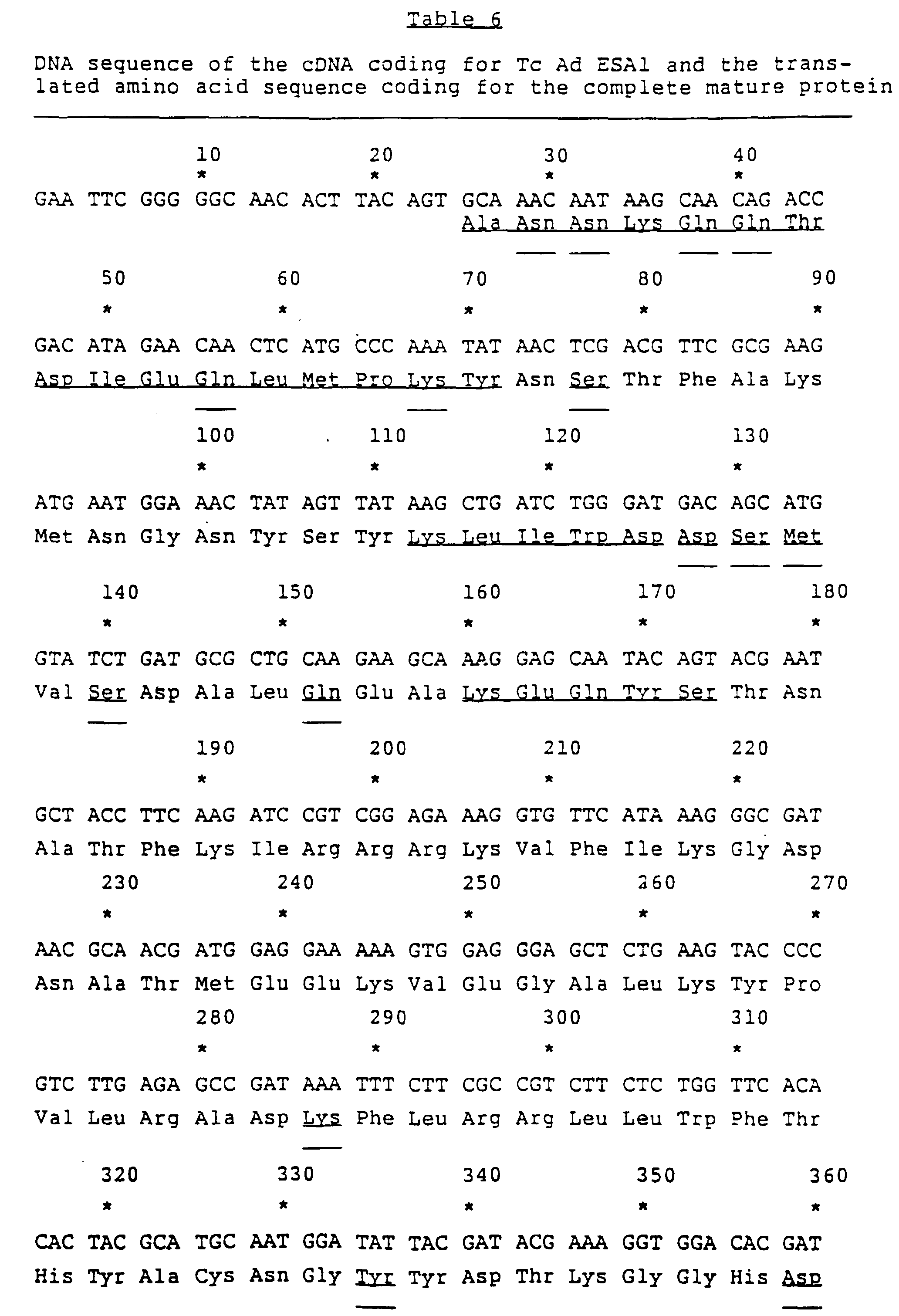

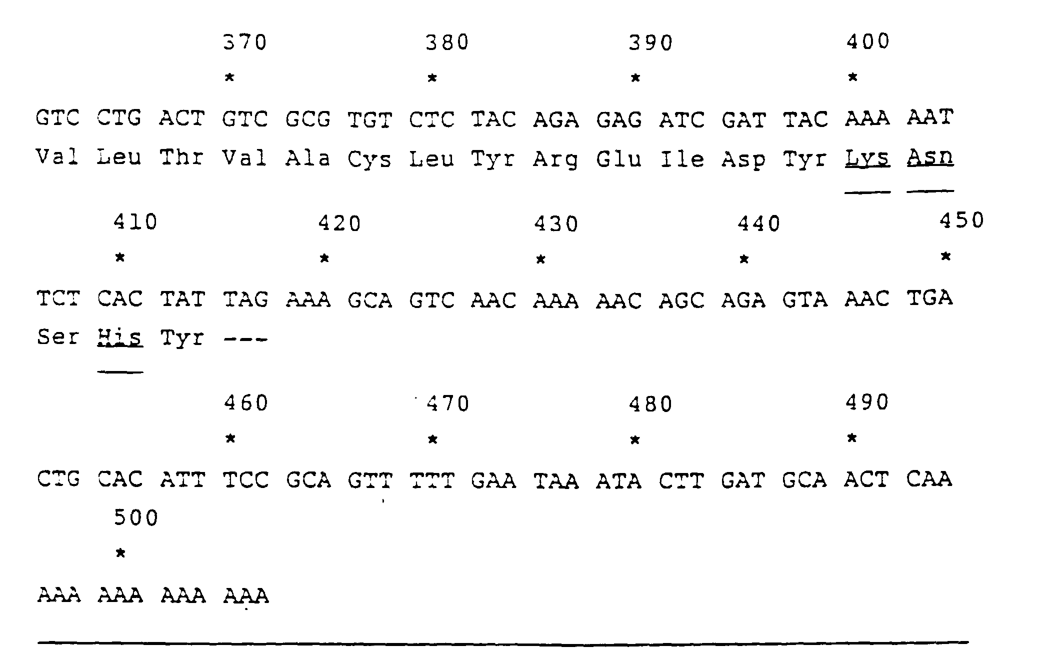

The DNA sequence of the clone coding for Tc Ad ESA4 is shown in Table 7. The DNA sequence contains for an open reading frame which codes for a protein of 92 amino acids, and contains only a single potential glycosylation site. E. coli strain TG1 transformed with an inhouse pBR322 based vector, pBTA503, containing the Tc Ad ESA4 gene has been given the inhouse reference number BTA 1690. The lack of binding to the lentil lectin column and the close agreement between the estimated molecular weight on SDS-PAGE and the predicted molecular weight based on the sequence suggests the protein is not glycosylated.

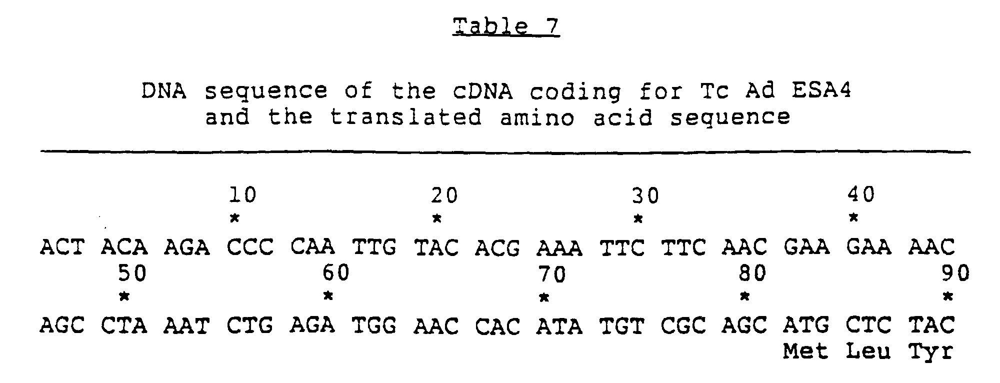

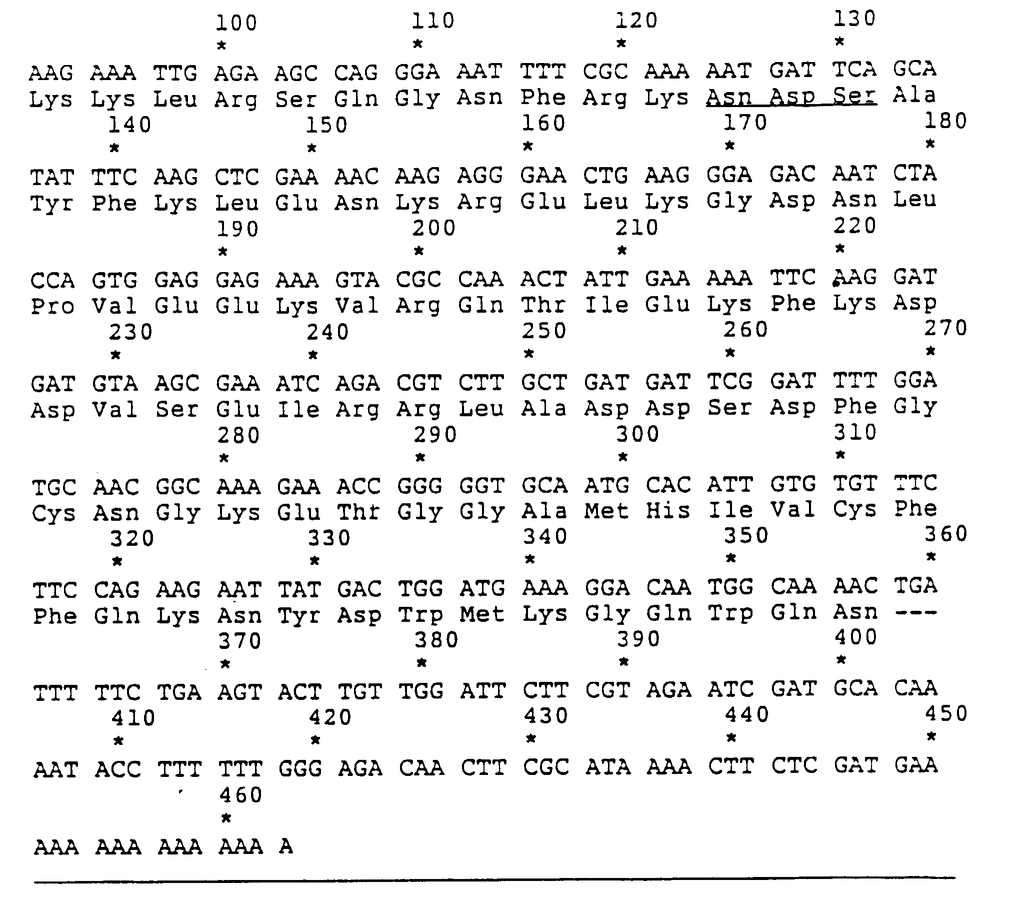

The DNA sequence of the partial clone coding for Tc Ad ESA3 is shown in Table 8. The DNA contains an open reading frame which codes for a peptide of 43 amino acids. The sequence corresponding to the N-terminal amino acid sequence from the natural protein is underlined. An E. coli strain DH5αF (BRL) transformed with plasmid pT₇T ₃(Pharmacia) containing the Tc Ad ESA3 gene fragment shown in Table 8 has been given the inhouse reference number BTA 1691.

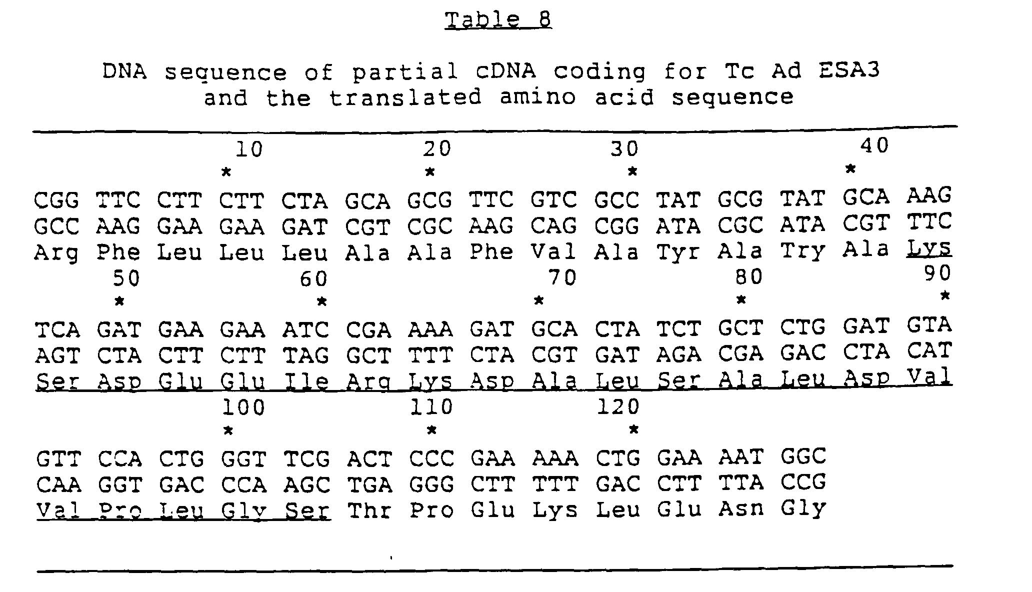

### Example 9

### Expression of Tc Ad ESA1 as a Tra T Fusion in E. coli

TraT is an outer membrane lipoprotein of certain strains of E. coli. We have cloned the gene coding for TraT obtained from the antibiotic resistance plasmid R100 (Ogata R.T. et al., 1982, J. Bact. 151 819-827) and have transferred this gene to a plasmid in which the expression of TraT is under the control of the leftward promoter (P_{L}) of the bacteriphage lambda. High levels of TraT can be obtained when the cells harbour the thermolabile repressor of P_{L}, λcI857 (Remaut E et al 1980 Gene 15 81-93) are incubated at 38-42^{o}C.

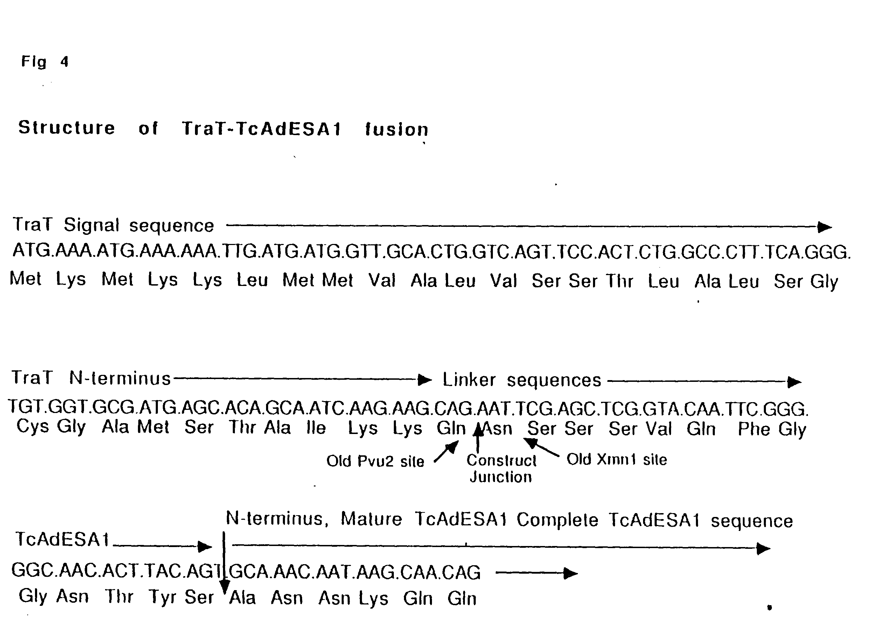

The gene coding for To Ad ESA1 has been fused to the 5' position of the coding region of TraT in such a way that the new gene codes for the first 30 amino acids of TraT (including the 20 amino acid long signal sequence) followed by some amino acids generated by restriction sites used for the DNA manipulations followed by the gene coding for Tc Ad ESA1 (Fig 4). Insertion into this position of the Tra-T gene was made possible by the creation of a PvuII restriction site at codons 31 and 32 of the TraT gene by site directed mutagenesis. The Tc Ad ESA1 gene was obtained as a 570bp XmnI (generated by cutting an EcoRI site, and filling with DNA polymerase I - Klenow fragment - and religating) to Hind III fragment.

In a suitable E. coli host, raising the temperature of a culture leads to the production of TraT-Tc Ad ESA1 fusion protein of apparent molecular weight 22kD at up to 50mgs per litre per OD₆₀₀.

The signal sequence may be cleaved from the fusion product (as is normally the case when TraT is produced in E. coli) if the level of expression does not exceed the processing capacity of the cell and the terminal cysteine may be further modified. When producing this TraT-immunogen fusion this modification may be advantageous as it may confer a self adjuvanting character to the protein (International Patent Application PCT/AU87/00107 Title: Immunopotentiation).

### Expression of Tc Ad ESA4 as a TraT Fusion

The gene coding for Tc Ad ESA4 has been fused to the 5' portion of the coding region of TraT in a manner identical to the Tc Ad ESA1-TraT construct. The whole of the coding region of Tc Ad ESA4 (92 amino acids) is expressed as a TraT Tc Ad ESA4 fusion under the control of the Lambda leftward promoter.

### Cloning into pYEUC114 and expression in Yeast

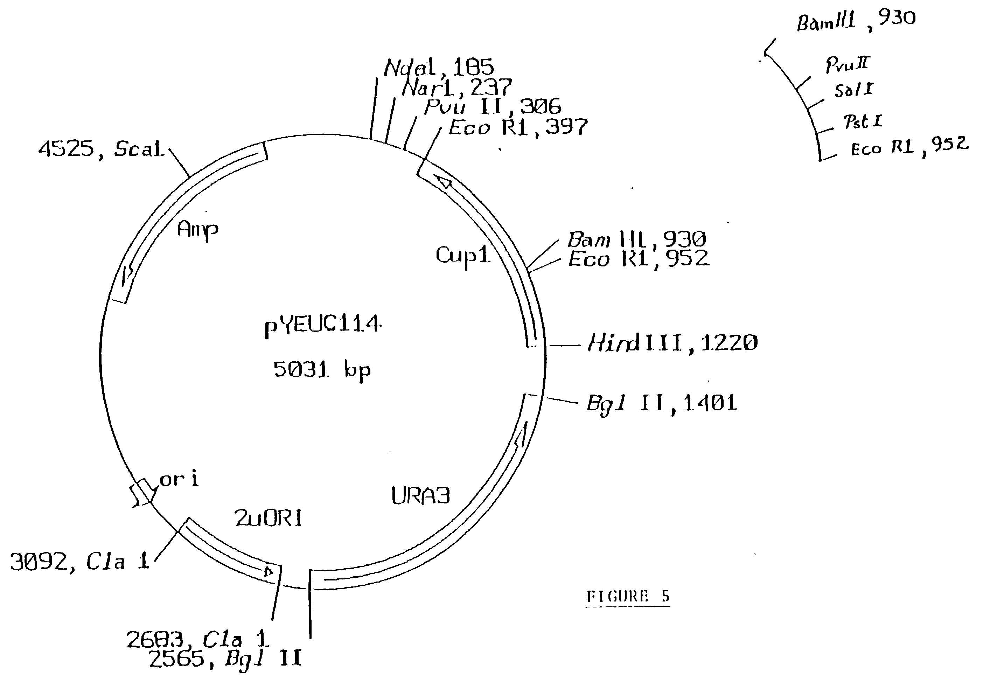

The cDNA fragment encoding Tc Ad ESA1 was inserted into a yeast expression vector, pYEUC114 (Fig 5), developed in the CSIRO Division of Biotechnology. This vector employs the Cup 1 gene (encoding metallothionine) of Saccharomyces cerevisiae. The accompanying promoter is inducible with copper when contained in yeast cells. The Cup 1 gene casette containing the copper-inducible Cup 1 promoter and a multi-cloning site is described in Australian Patent Application No. 15845/88 and in Macreadie et al, Plasmid 2, 147-150. The EcoR1 fragment containing the (previously described) Tc Ad ESA1 cDNA was inserted into pYEUC114 replacing most of the Cup 1 coding sequence. This results in the synthesis of a fusion protein consisting of 4 amino acids from the N-terminus of metallothionine followed by the sequence shown in Table 6. Saccharomyces cerevisiae cells (strain CL13-ABSY86, [α, ΔUra3 leu2 his pra1 prb1 prc1 cps1]) carrying the recombinant plasmid (pYEUC30B4E) were grown in minimal medium containing histidine and leucine. To induce expression of Tc Ad ESA1, copper sulphate was added to the culture medium to 0.5mM. After 2 hours in the presence of copper, the cells were harvested, treated with Zymolyase to remove the yeast outer cell wall and then examined by SDS-PAGE and western blotting. The recombinant plasmid containing Tc Ad ESA1 encoding DNA was named pYEUC30B4E

### Example 10

### Purification of recombinant antigens from bacteria and yeast

The antigens expressed by recombinant E. coli cells can be purified for vaccination trials. By means of example the following is an illustration of how the Tc Ad ESA1 is isolated.

Bacterial cells containing the recombinant plasmid described in Example 9 are grown in a suitable medium at 28^{o}C and the expression of Tc Ad EAS1 is induced by increasing the temperature to 42^{o}C and incubation the cultures at that temperature for 4-6 hours. Cells are recovered from cultures by centrifugation at 10,000 xg for 10 mins at 4^{o}C. The pellet is then resuspended in a suitable buffer such as 50 mM Tris-HCl, 10mM EDTA, 50 mM NaCl, pH 8.0 and cells pelleted by centrifugation as before. The washed pellet is resuspended in a buffer such as 50 mM Tris-HCl, 1 mM EDTA, 5 mM DTT, 0.1 mM PMSF, pH 8.0 and homogenised in Marton-Gaulin Homogeniser, 6 passes at 9000 psi. The cell homogenate is then centrifuged at 20,000 xg for 20 min at 4^{o}C to collect the dense inclusion bodies which contain the recombinant antigen. The supernatant is decanted off and discarded and the pellet is resuspended in a solution suitable for solubilising the proteins in the inclusion bodies such as 8 M Urea, 100 mM NaPi, 1 mM EDTA, 40 mM DTT, pH 8.5 and incubated at 37^{o}C for 4 hours with stirring. The solubilised antigen can be recovered by passing the solution through a "Diaflo" Amicon YM30 membrane followed by concentration of the eluant on a "Diaflo" Amicon YM10 membrane. The retenate can then be adjusted to pH 3.0 by addition of phosphoric acid, diluted 1:1 with 8M Urea to reduce the Na⁺ concentration to 50 mM and passed over a column of S-sepharose "Fast Flow" equilibrated with 8 M Urea, 50 mM NaPi, 5 mM EDTA, 5 mM DTT, pH 3.0. The recombinant antigen is eluted off the column with a 50 - 400 mM NaPi gradient. Fractions containing the 21 kD recombinant Tc Ad ESA antigen are pooled and concentrated on a "Diaflo" Amicon YM10 membrane. This concentrate is then made 0.1% with respect to SDS and dialysed in a 1000 D cut off dialysis sac against 8 M Urea, 50 mM NaPi, 2mM DTT, 0.1% SDS, pH 8.5 to reduce the Na⁺ concentration to 50 mM and increase the pH to 8.5. The antigen can then be dialysed against a solution containing 150 mM NaCl, 10 mM Tris-HCl, 0.006 mM Oxidised Glutathione, 0.06 mM Reduced Glutathione, 0.1% SDS, pH 8.5, at room temperature for 24 hours and finally against a solution containing 150 mM NaCl, 10 mM Tris-HCl, 0.1% SDS, pH 7.4, at room temperature for 24 hours. The antigen recovered from the dialysis sac can be sterilised by filtration through a 0.22 µm filter prior to formulation in a suitable adjuvant prior to vaccination of host animals.

A similar approach can be taken to purify the other recombinant antigens according to this specification although the details of the purification protocols will differ with each antigen.

### Preparation of Recombinant Tc Ad ESA1 from Yeast

Yeast cells carrying pYEUC30B4E were grown for 2 days in minimal medium containing histidine and leucine. The cells were then placed in fresh medium, incubated for a further 2hrs and then copper sulphate was added to 0.5mM. Incubations was continued for a further 2hrs whereupon the cells were harvested by centrifugation and lysed using the Braun cell homogenizer according to the manufacturer's instructions. Briefly the cells are broken by shaking with glass beads at high speed. The glass beads are allowed to settle out under gravity and the cell lysate collected. The crude lysate was centrifuged at 15,000 rpm and the resulting supernatant and pellet examined for the presence of Tc Ad ESA1 protein. The latter was found exclusively in the 15krpm pellet. This pellet was subsequently dissolved in 50mM ammonium bicarbonate solution containing 8M urea, 2% SDS, 10mM EDTA and 2% mercaptoethanol. This crude material was then fractionated using a Sephadex G75 column run in 50mM ammonium bicarbonate solution containing 1mM EDTA, 0.1% SDS and 0.1% mercaptoethanol. Fractions containing material with the molecular weight expected of Tc Ad ESA1 (non-glycosylated) and reacting with an anti-serum (R45) raised in rabbits against Tc Ad ESA1 from adult parasites, were pooled and used in subsequent vaccination trials.

### Example 11

### Host-Protection Using Recombinant Tc Ad ESA1 produced by yeast

| #295 | Worm numbers ±SD | % Protection |
|---|---|---|
| Controls | 413±299 | |
| Vaccinates | 275±166 | 33 |

Guinea pigs were vaccinated with a preparation of recombinant Tc Ad ESA1 and challenged with T. colubriformis as described above. It should be noted that the worm numbers in the control animals were uncharacteristically low and scattered in this particular experiment. In previous instances where this has occurred (see e.g. Table 3, Experiment 257) repeat experiments have resulted in increased levels of protection being observed (see e.g. Table 3, Experiment 236).

### Example 12

### Extension to other Parasites

The Tc Ad ESA antigens produced by recombinant DNA technology are capable of inducing a protective immune response against T. colubriformis infestation in vaccinated animals. It is possible that this immune response may also provide protection against other species of parasitic nematodes such as those cited elsewhere in this specification, but it is more likely that the other species of parasitic nematodes express proteins which are related but not identical to the Tc Ad ESA antigens. For most species of parasitic nematodes, it is not practical to obtain sufficient parasite material to purify these components and identify their structure in preparation for cloning the gene from those parasites and testing the protective potential of the components. In these cases, the only means by which the related antigens can be tested is to use recombinant DNA methods to isolate the gene coding for the related proteins and to express the related proteins in recombinant organisms, purify the related proteins from those recombinant organisms and vaccinate animals and challenge them with the other species of parasitic nematodes. Even in the cases where it is possible to obtain sufficient parasite material to purify antigens, the above approach using molecular biology to clone genes coding for protective antigens related to the Tc Ad ESA antigen genes is a preferable approach to developing vaccines. To demonstrate that this approach is feasible, the following example demonstrates that there are genes that are related to Tc Ad ESA1 and 4 in two species of parasitic nematodes other than T. colubriformis, namely Haemonchus contortus which is an abomasal parasite of sheep and goats in particular and Dirofilaria immitis which is a parasitise of dogs and cats.

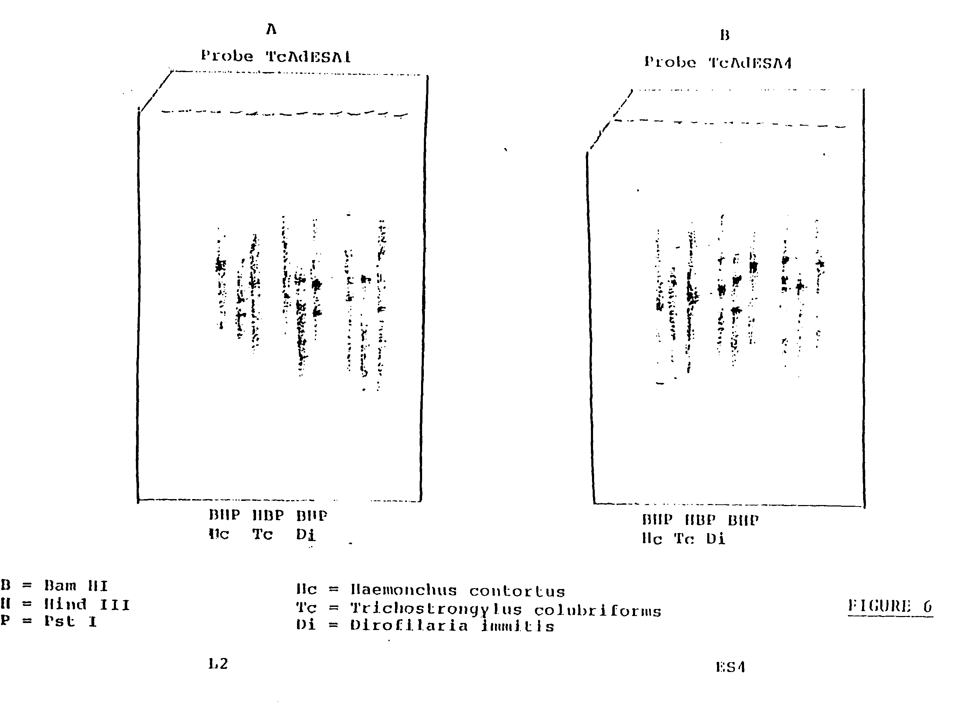

Genomic DNA was purified from the three species of parasites by the method described by Herrmann and Frischauf 1987. 1µg of each DNA preparation was digested with each of the restriction enzymes BamHI, PstI and HindIII (Promega). The digested DNA together with appropriate size markers was electrophoresed in 0.4% agarose gels to separate the DNA fragments according to their size. After staining the gels in ethidium bromide and photographing the DNA, the DNA in the gels was transferred to positively charged nylon membranes by alkaline transfer and the membranes prepared for hybridisation as recommended by the manufacturers (Amersham). Fragments of DNA coding for Tc Ad ESA1 and 4 were labeled with ³²P by the random labeling method described in the kit sold by Boehringer-Manheim. The filters were then incubated for 20 hours at 42°C in a solution of 5xSSPE, 5x Denhardts solution, 0.5% SDS and 20 µg/ml of denatured Herring sperm DNA (see Maniatis et al 1982) containing 10⁶ cpm/ml of the radioactive Tc Ad ESA DNA. The filters were then washed to remove any non-specifically bound DNA and exposed to X-ray film. Fig. 6 demonstrates that there are specific DNA sequences in both H contortus and D immitis DNA which hybridise to the Tc Ad ESA DNA fragments. This demonstrates that these DNA sequences could be cloned from genomic DNA libraries or from cDNA libraries or prepared by other recombinant DNA techniques such as the polymerase chain reaction from these species of parasite and by extension from any other species of parasitic nematode and recombinant organisms could be constructed which synthesise these related genes for use in vaccines against the other species of parasitic nematodes.

### Example 13

### Scale up of Manufacturing for Commercial Vaccines

The production and purification techniques so far described are carried out at laboratory scale. For commercial production of the antigens of the invention, large scale fermentation of transformed hosts is required.

The large scale fermentations are performed according to standard techniques, the particular techniques selected being appropriate to the transformed host used for production of the antigen.

### INDUSTRIAL APPLICATIONS

The invention provides antigens which can be used as an effective vaccine for protection against parasitism of animals by parasitic nematodes, particularly Trichostrongylus colubriformis and Haemonchus contortus.

Antibodies raised against the purified antigens isolated from Trichostrongylus colubriformis and the DNA sequences coding for these proteins can be used to identify the related polypeptides and genes coding for the antigens from species of parasitic nematode other than Trichostrongylus colubriformis. The same DNA sequences and antibodies can be used to identify related antigens and genes coding for those proteins in a range of other species of nematode which are parasitic to man and domestic animals and it is anticipated that these proteins will provide effective vaccines against parasitism by those species of nematode whether isolated from the parasite itself or produced by recombinant DNA technology. Species of parasites and hosts they may infect include for example:

Trichinella spiralis or Ancylostoma caninum infections of man, Strongylus vulgaris infections of horses, Trichostrongylus colubriformis infections of sheep, Haemonchus contortus infections of goats, Ostertagia ostertagi infections of cattle, Ascaris suum or Trichinella spiralis infections of pigs, Toxascaris leonina or Uncinaria stenocephala infections of cats and Ancylostoma caninum or Trichuris vulpis infections of dogs as well as infections of the circulatory system of man by larvae of Toxocara spp and of the circulatory system of dogs by Dirofilaria immitis as well as infections of the circulatory system, urogenital system, respiratory system, skin and subcutaneous tissues of these and other species of animal. It should be noted that this list is by no means complete.

## Claims

1. An antigen comprising an excretory/secretory protein derived from a parasitic stage of Trichostrongylos colubriformis and being capable of conferring protective passive immunity on a host against infection by a parasitic nematode, characterised in that it has:
an approximate molecular weight of 11kD as estimated by SDS-PAGE and comprising the amino acid sequence: 
Met Leu Tyr Lys Lys Leu Arg Ser Gln Gly Asn Phe Arg Lys
Asn Asp Ser Ala Tyr Phe Lys Leu Glu Asn Lys Arg Glu Leu
Lys Gly Asp Asn Leu Pro Val Glu Glu Lys Val Arg Gln Thr
Ile Glu Lys Phe Lys Asp Asp Val Ser Glu Ile Arg Arg Leu
Ala Asp Asp Ser Asp Phe Gly Cys Asn Gly Lys Glu Thr Gly
Gly Ala Met His Ile Val Cys Phe Phe Gln Lys Asn Tyr Asp
Trp Met Lys Gly Gln Trp Gln Asn
an approximate molecular weight of 17kD as estimated by SDS-PAGE and comprising the amino acid sequence: 
Arg Phe Leu Leu Leu Ala Ala Phe Val Ala Tyr Ala Try Ala
Lys Ser Asp Glu Glu Ile Arg Lys Asp Ala Leu Ser Ala Leu
Asp Val Val Pro Leu Gly Ser Thr Pro Glu Lys Leu Glu Asn Gly
an approximate molecular weight of 30kD as estimated by SDS-PAGE and the amino acid sequence: 
Ala Asn Asn Lys Gln Gln Thr Asp Ile Glu Gln Leu Met Pro
Lys Tyr Asn Ser Thr Phe Ala Lys Met Asn Gly Asn Tyr Ser
Tyr Lys Leu Ile Trp Asp Asp Ser Met Val Ser Asp Ala Leu
Gln Glu Ala Lys Glu Gln Tyr Ser Thr Asn Ala Thr Phe Lys
Ile Arg Arg Arg Lys Val Phe Ile Lys Gly Asp Asn Ala Thr
Met Glu Glu Lys Val Glu Gly Ala Leu Lys Tyr Pro Val Leu
Arg Ala Asp Lys Phe Leu Arg Arg Leu Leu Trp Phe Thr His
Tyr Ala Cys Asn Gly Tyr Tyr Asp Thr Lys Gly Gly His Asp
Val Leu Thr Val Ala Cys Leu Tyr Arg Glu Ile Asp Tyr Lys
Asn Ser His Tyr
an approximate molecular weight of 37kD as estimated by SDS-PAGE and comprising the amino acid sequences: Lys Ser Ile Leu Arg Leu and 
Lys Val Ile Pro Xaa Asn Pro Pro Ile Lys Asp Thr
Pro,
or an approximate molecular weight of 81kD as measured by SDS-PAGE and comprising the amino terminal amino acid sequence Ser Xaa Ser Leu Lys Asp;
or comprising a part, an analogue, homologue or combinations thereof of said excretory/secretory protein.

2. An antigen comprising an excretory/secretory protein according to Claim 1 having an approximate molecular weight of 11 kD as estimated by SDS-PAGE.

3. An antigen comprising an excretory/secretory protein according to Claim 1 having an approximate molecular weight of 17 kD as estimated by SDS-PAGE.

4. An antigen comprising an excretory/secretory protein according to Claim 1 having an approximate molecular weight of 30 kD as estimated by SDS-PAGE.

5. An antigen comprising an excretory/secretory protein according to Claim 1 having an approximate molecular weight of 37 kD as estimated by SDS-PAGE.

6. An antigen comprising an excretory/secretory protein according to Claim 1 having an approximate molecular weight of 81 kD as estimated by SDS-PAGE.

7. A nucleotide sequence encoding the amino acid sequence of an antigen according to any one of Claims 1 to 6.

8. A nucleotide sequence according to Claim 7, wherein the nucleotide sequence is a DNA sequence.

9. A DNA sequence according to Claim 8 comprising:

GAA TTC GGG GGC AAC ACT TAC AGT GCA AAC AAT AAG CAA CAG ACC
GAC ATA GAA CAA CTC ATG CCC AAA TAT AAC TCG ACG TTC GCG AAG
ATG AAT GGA AAC TAT AGT TAT AAG CTG ATC TGG GAT GAC AGC ATG
GTA TCT GAT GCG CTG CAA GAA GCA AAG GAG CAA TAC AGT ACG AAT
GCT ACC TTC AAG ATC CGT CGG AGA AAG GTG TTC ATA AAG GGC GAT
AAC GCA ACG ATG GAG GAA AAA GTG GAG GGA GCT CTG AAG TAC CCC
GTC TTG AGA GCC GAT AAA TTT CTT CGC CGT CTT CTC TGG TTC ACA
CAC TAC GCA TGC AAT GGA TAT TAC GAT ACG AAA GGT GGA CAC GAT
GTC CTG ACT GTC GCG TGT CTC TAC AGA GAG ATC GAT TAC AAA AAT
TCT CAC TAT TAG AAA GCA GTC AAC AAA AAC AGC AGA GTA AAC TGA
CTG CAC ATT TCC GCA GTT TTT GAA TAA ATA CTT GAT GCA ACT CAA
AAA AAA AAA AAA

10. A DNA sequence according to Claim 8 comprising:

ACT ACA AGA CCC CAA TTG TAC ACG AAA TTC TTC AAC GAA GAA AAC
AGC CTA AAT CTG AGA TGG AAC CAC ATA TGT CGC AGC ATG CTC TAC
AAG AAA TTG AGA AGC CAG GGA AAT TTT CGC AAA AAT GAT TCA GCA
TAT TTC AAG CTC GAA AAC AAG AGG GAA CTG AAG GGA GAC AAT CTA
CCA GTG GAG GAG AAA GTA CGC CAA ACT ATT GAA AAA TTC AAG GAT
GAT GTA AGC GAA ATC AGA CGT CTT GCT GAT GAT TCG GAT TTT GGA
TGC AAC GGC AAA GAA ACC GGG GGT GCA ATG CAC ATT GTG TGT TTC
TTC CAG AAG AAT TAT GAC TGG ATG AAA GGA CAA TGG CAA AAC TGA
TTT TTC TGA AGT ACT TGT TGG ATT CTT CGT AGA ATC GAT GCA CAA
AAT ACC TTT TTT GGG AGA CAA CTT CGC ATA AAA CTT CTC GAT GAA
AAA AAA AAA AAA A

11. A DNA sequence according to Claim 8 comprising:

[?]GG TTC CTT CTT CTA GCA GCG TTC GTC GCC TAT GCG TAT GCA AAG
TCA GAT GAA GAA ATC CGA AAA GAT GCA CTA TCT GCT CTG GAT GTA
GTT CCA CTG GGT TCG ACT CCC GAA AAA CTG GAA AAT GGC

12. A process for selecting a DNA or RNA sequence coding for an antigen according to any one of Claims 1 to 6, which process comprises providing one or more DNA or RNA sequences, determining which of the sequence hybridizes with a DNA or RNA sequence known to code for an antigen according to any one of Claims 1 to 6, or providing an anti-serum to the antigen and identifying host-vector combinations that express the antigen.

13. A recombinant DNA molecule comprising a DNA sequence according to any one of Claims 8 to 11 and vector DNA.

14. A recombinant DNA molecule according to Claim 13 additionally comprising an expression control sequence operatively linked to the DNA sequence.

15. A recombinant DNA molecule according to Claim 14, wherein the expression control sequence is selected from: the β-galactosidase gene of E.coli, the tryptophan operon, the Tra-T gene of E.coli, the leftward promoter of bacteriophage lambda, the tac promoter, the Cup 1 promoter and the SV40 early promoter.

16. A recombinant DNA molecule according to any one of Claims 13 to 15, wherein the vector DNA is plasmid DNA.

17. A recombinant DNA molecule according to Claim 13 or Claim 14, wherein the vector DNA is bacteriophage DNA.

18. A recombinant DNA molecule according to Claim 17, wherein the bacteriophage DNA is selected from: bacteriophage lambda and derivatives thereof, including lambda gt10 and lambda gt11.

19. A fused gene comprising a promoter, a translation start signal and a DNA sequence according to any one of Claims 8 to 11.

20. A process for the preparation of a recombinant DNA molecule according to any one of Claims 13 to 18 which process comprises providing a DNA insert comprising a DNA sequence according to any one of Claims 8 to 11 and introducing the DNA insert into vector DNA.

21. A process according to Claim 20, wherein the DNA inserted is introduced into the cloning vector in correct spacing and correct reading frame with respect to an expression control sequence.

22. A transformed host, transformed with at least one recombinant DNA molecule according to any one of Claims 13 to 18.

23. A transformed host according to Claim 22, wherein the host is capable of expressing an antigen according to any one of Claims 1 to 6.

24. A transformed host according to Claim 22 or Claim 23, wherein the host is a bacterial cell, yeast, including Saccharomyces cerevisiae strain CL13-ABSY86, other fungus, vertebrate cell, insect cell, a plant cell, human cell, human tissue cell live virus such as vaccinia or baculovirus, or a whole eukaryotic organism.

25. A transformed host according to any one of Claims 22 to 24, wherein the host is E.coli, an enteric organism other than E.coli, a Pseudomonas or Bacillus species.

26. A transformed host according to Claim 25 wherein the host is an E.coli K12 derivative selected from JM109 and Y1090.

27. A process for transforming a host to provide a transformed host according to any one of Claims 22 to 26 which process comprising providing a host, making the host competent for transformation, and introducing into the host a recombinant DNA molecule according to any one of Claims 13 to 18.

28. An expression product of a transformed host according to any one of Claims 22 to 26 which product comprises an antigen according to any one of Claims 1 to 6.

29. An expression product according to Claim 28 in purified form.

30. An expression product according to Claim 28 or Claim 29 comprising a first polypeptide sequence homologous to the host and a second polypeptide sequence which is an amino acid sequence coding for an antigen according to any one of Claims 1 to 6.

31. An expression product according to Claim 30, wherein the first polypeptide sequence is all or part of β-galactosidase or Tra-T and the host E.coli.

32. A process for the biosynthesis of a proteinaceous product comprising an antigen according to any one of Claims 1 to 6 which process comprises: transforming a host with a recombinant DNA molecule according to any one of Claims 14 to 18 so that the host is capable of expressing a proteinaceous product which includes an antigen according to any one of Claims 1 to 6; culturing the host to obtain expression; and collecting the proteinaceous product.

33. A vaccine comprising an effective amount of one or more antigens according to any one of Claims 1 to 6, expression products according to any one of Claims 28 to 31, together with a pharmaceutically acceptable carrier, diluent, excipient and/or adjuvant.

34. A vaccine according to Claim 52 suitable for oral administration or in injectable form.

35. An antibody prepared as a result of vaccination of a host by administration of one or more antigens according to any one of Claims 1 to 6, expression products according to any one of Claims 28 to 31 and/or vaccines according to any one of Claims 33 and 34 to the host.

36. A process for the preparation of an antigen according to any one of Claims 1 to 6 which process comprises: collecting excretory/secretory fluids from a parasitic stage of a parasitic nematode species; fractionating the fluids by lentil lectin chromatography with methyl mannoside as eluent; collecting the bound and unbound fractions; further fractionating by SDS-gel electrophoresis; and electroeluting the antigen.

37. A process for the preparation of a fused gene according to Claim 24 which process comprises providing a promoter, a translation start signal and a DNA sequence according to any one of Claims 8 to 11 and operatively linking the promoter, translation start signal and DNA sequence.

38. A process for the preparation of a vaccine according to any one of Claims 33 and 34 which process comprises admixing an effective amount of at least one antigen according to any one of Claims 1 to 6 and/or expression products according to any one of Claims 28 to 31 with a pharmaceutically acceptable carrier, diluent, excipient and/or adjuvant.

39. A process for the preparation of an antibody according to Claim 35 which process comprises:
immunising an immunoresponsive host with an effective amount of antigen according to any one of Claims 1 to 6, an expression product according to any one of Claims 28 to 31, or a vaccine according to any one of Claims 52 and 53.

40. An antigen according to any one of Claims 1 to 6, expression product according to any one of Claims 28 to 31, and/or vaccines according to any one of Claims 33 or 34 for use in a method of treatment of infection by a parasitic nematode.

41. An antibody according to Claim 35, for use in a method of passively protecting a host against infection by a parasitic nematode.

## Patentansprüche

1. Antigen, enthaltend ein exkretorisches/sekretorisches Protein, das von einer parasitischen Stufe von Trichostrongylos colubriformis stammt und einem Wirt eine schützende passive Immunität gegen eine Infektion durch parasitische Nematoden verleihen kann,
**dadurch gekennzeichnet**, daß es ein ungefähres Molekulargewicht von 11 kD gemäß SDS-PAGE hat und die folgende Aminosäuresequenz aufweist: 
Met Leu Tyr Lys Lys Leu Arg Ser Gln Gly Asn Phe Arg Lys
Asn Asp Ser Ala Tyr Phe Lys Leu Glu Asn Lys Arg Glu Leu
Lys Gly Asp Asn Leu Pro Val Glu Glu Lys Val Arg Gln Thr
Ile Glu Lys Phe Lys Asp Asp Val Ser Glu Ile Arg Arg Leu
Ala Asp Asp Ser Asp Phe Gly Cys Asn Gly Lys Glu Thr Gly
Gly Ala Met His Ile Val Cys Phe Phe Gln Lys Asn Tyr Asp
Trp Met Lys Gly Gln Trp Gln Asn
 ein ungefähres Molekulargewicht von 17 kD gemäß SDS-PAGE hat und die folgende Aminosäuresequenz aufweist: 
Arg Phe Leu Leu Leu Ala Ala Phe Val Ala Tyr Ala Try Ala
Lys Ser Asp Glu Glu Ile Arg Lys Asp Ala Leu Ser Ala Leu
Asp Val Val Pro Leu Gly Ser Thr Pro Glu Lys Leu Glu Asn Gly
 ein ungefähres Molekulargewicht von 30 kD gemäß SDS-PAGE hat und die folgende Aminosäuresequenz aufweist: 
Ala Asn Asn Lys Gln Gln Thr Asp Ile Glu Gln Leu Met Pro
Lys Tyr Asn Ser Thr Phe Ala Lys Met Asn Gly Asn Tyr Ser
Tyr Lys Leu Ile Trp Asp Asp Ser Met Val Ser Asp Ala Leu
Gln Glu Ala Lys Glu Gln Tyr Ser Thr Asn Ala Thr Phe Lys
Ile Arg Arg Arg Lys Val Phe Ile Lys Gly Asp Asn Ala Thr
Met Glu Glu Lys Val Glu Gly Ala Leu Lys Tyr Pro Val Leu
Arg Ala Asp Lys Phe Leu Arg Arg Leu Leu Trp Phe Thr His
Tyr Ala Cys Asn Gly Tyr Tyr Asp Thr Lys Gly Gly His Asp
Val Leu Thr Val Ala Cys Leu Tyr Arg Glu Ile Asp Tyr Lys
Asn Ser His Tyr
 ein ungefähres Molekulargewicht von 37 kD gemäß SDS-PAGE hat und die folgenden Aminosäuresequenzen aufweist: Lys Ser Ile Leu Arg Leu und Lys Val Ile Pro Xaa Asn Pro Pro Ile Lys Asp Thr Pro, oder ein ungefähres Molekulargewicht von 81 kD, gemessen mit SDS-PAGE hat und die aminoterminale Aminosäuresequenz Ser Xaa Ser Leu Lys Asp hat oder ein Teil des exkretorischen/sekretorischen Proteins, ein analoges, homologes exkretorisches/sekretorisches Protein oder Kombinationen davon.

2. Antigen, umfassend ein exkretorisches/sekretorisches Protein nach Anspruch 1 mit einem ungefähren Molekulargewicht von 11 kD gemäß SDS-PAGE.

3. Antigen, umfassend ein exkretorisches/sekretorisches Protein nach Anspruch 1 mit einem ungefähren Molekulargewicht von 17 kD gemäß SDS-PAGE.

4. Antigen, umfassend ein exkretorisches/sekretorisches Protein nach Anspruch 1 mit einem ungefähren Molekulargewicht von 30 kD gemäß SDS-PAGE.

5. Antigen, umfassend ein exkretorisches/sekretorisches Protein nach Anspruch 1 mit einem ungefähren Molekulargewicht von 37 kD gemäß SDS-PAGE.

6. Antigen, umfassend ein exkretorisches/sekretorisches Protein nach Anspruch 1 mit einem ungefähren Molekulargewicht von 81 kD gemäß SDS-PAGE.

7. Nucleotidsequenz, die die Aminosäuresequenz eines Antigens nach einem der Ansprüche 1 bis 6 kodiert.

8. Nucleotidsequenz nach Anspruch 7, worin die Nucleotidsequenz eine DNA-Sequenz ist.

9. DNA-Sequenz nach Anspruch 8 umfassend:

GAA TTC GGG GGC AAC ACT TAC AGT GCA AAC AAT AAG CAA CAG ACC
GAC ATA GAA CAA CTC ATG CCC AAA TAT AAC TCG ACG TTC GCG AAG
ATG AAT GGA AAC TAT AGT TAT AAG CTG ATC TGG GAT GAC AGC ATG
GTA TCT GAT GCG CTG CAA GAA GCA AAG GAG CAA TAC AGT ACG AAT
GCT ACC TTC AAG ATC CGT CGG AGA AAG GTG TTC ATA AAG GGC GAT
AAC GCA ACG ATG GAG GAA AAA GTG GAG GGA GCT CTG AAG TAC CCC
GTC TTG AGA GCC GAT AAA TTT CTT CGC CGT CTT CTC TGG TTC ACA
CAC TAC GCA TGC AAT GGA TAT TAC GAT ACG AAA GGT GGA CAC GAT
GTC CTG ACT GTC GCG TGT CTC TAC AGA GAG ATC GAT TAC AAA AAT
TCT CAC TAT TAG AAA GCA GTC AAC AAA AAC AGC AGA GTA AAC TGA
CTG CAC ATT TCC GCA GTT TTT GAA TAA ATA CTT GAT GCA ACT CAA
AAA AAA AAA AAA

10. DNA-Sequenz nach Anspruch 8, umfassend:

ACT ACA AGA CCC CAA TTG TAC ACG AAA TTC TTC AAC GAA GAA AAC
AGC CTA AAT CTG AGA TGG AAC CAC ATA TGT CGC AGC ATG CTC TAC
AAG AAA TTG AGA AGC CAG GGA AAT TTT CGC AAA AAT GAT TCA GCA
TAT TTC AAG CTC GAA AAC AAG AGG GAA CTG AAG GGA GAC AAT CTA
CCA GTG GAG GAG AAA GTA CGC CAA ACT ATT GAA AAA TTC AAG GAT
GAT GTA AGC GAA ATC AGA CGT CTT GCT GAT GAT TCG GAT TTT GGA
TGC AAC GGC AAA GAA ACC GGG GGT GCA ATG CAC ATT GTG TGT TTC
TTC CAG AAG AAT TAT GAC TGG ATG AAA GGA CAA TGG CAA AAC TGA
TTT TTC TGA AGT ACT TGT TGG ATT CTT CGT AGA ATC GAT GCA CAA
AAT ACC TTT TTT GGG AGA CAA CTT CGC ATA AAA CTT CTC GAT GAA
AAA AAA AAA AAA A

11. DNA-Sequenz nach Anspruch 8, umfassend:

[?]GG TTC CTT CTT CTA GCA GCG TTC GTC GCC TAT GCG TAT GCA AAG
TCA GAT GAA GAA ATC CGA AAA GAT GCA CTA TCT GCT CTG GAT GTA
GTT CCA CTG GGT TCG ACT CCC GAA AAA CTG GAA AAT GGC

12. Verfahren zum Selektieren einer DNA- oder RNA-Sequenz, die ein Antigen nach einem der Ansprüche 1 bis 6 kodiert, wobei das Verfahren umfaßt, daß man ein oder mehrere DNA- oder RNA-Sequenzen bereitstellt, feststellt, welche der Sequenzen mit einer DNA- oder RNA-Sequenz hybridisiert, von der bekannt ist, daß sie ein Antigen nach einem der Ansprüche 1 bis 6 kodiert, oder ein Antiserum gegen das Antigen bereitstellt und Wirt-Vektor-Kombinationen identifiziert, die das Antigen exprimieren.

13. Rekombinantes DNA-Molekül umfassend eine DNA-Sequenz nach einem der Ansprüche 8 bis 11 und Vektor-DNA.

14. Rekombinantes DNA-Molekül nach Anspruch 13, das zusätzlich eine Expressions-Kontrollsequenz, die operativ mit der DNA-Sequenz verbunden ist, umfaßt.

15. Rekombinantes DNA-Molekül nach Anspruch 14, worin die Expressions-Kontrollsequenz ausgewählt ist aus dem β-Galactosidase-Gen von E. coli, dem Tryptophan-Operon, dem Tra-T-Gen von E. coli, dem linken Promotor des Bacteriophagen Lambda, dem tac-Promotor, dem Cup 1-Promotor und dem frühen SV40-Promotor.

16. Rekombinantes DNA-Molekül nach einem der Ansprüche 13 bis 15, worin die Vektor-DNA Plasmid-DNA ist.

17. Rekombinantes DNA-Molekül nach Anspruch 13 oder Anspruch 14, worin die Vektor-DNA Bakteriophagen-DNA ist.

18. Rekombinantes DNA-Molekül nach Anspruch 17, worin die Bakteriophagen-DNA ausgewählt ist aus: Bakteriophagen Lambda und Derivaten davon, einschließlich lambda gt10 und lambda gt11.

19. Fusioniertes Gen, umfassend einen Promotor, ein Translations-Startsignal und eine DNA-Sequenz nach einem der Ansprüche 8 bis 11.

20. Verfahren zur Herstellung eines rekombinanten DNA-Moleküls nach einem der Ansprüche 13 bis 18, wobei das Verfahren umfaßt, daß man ein DNA-Insert bereitstellt, das eine DNA-Sequenz nach einem der Ansprüche 8 bis 11 umfaßt und das DNA-Insert in Vektor-DNA einsetzt.

21. Verfahren nach Anspruch 20, worin die eingesetzte DNA in den Klonierungsvektor im richtigen Abstand und richtigen Leserahmen im Hinblick auf eine Expressions-Kontrollsequenz eingeführt wird.

22. Transformierter Wirt, transformiert mit mindestens einem rekombinanten DNA-Molekül nach einem der Ansprüche 13 bis 18.

23. Transformierter Wirt nach Anspruch 22, worin der Wirt das Antigen nach einem der Ansprüche 1 bis 6 exprimieren kann.

24. Transformierter Wirt nach Anspruch 22 oder Anspruch 23, worin der Wirt eine Bakterienzelle, eine Hefe, einschließlich dem Saccharomyces cerevisiae-Stamm CL13-ABSY86, ein anderer Pilz, eine Wirbeltierzelle, eine Insektenzelle, eine Pflanzenzelle, eine menschliche Zelle, eine menschliche Gewebezelle, ein lebender Virus, wie z.B. Vaccinia oder Baculovirus, oder ein ganzer eukaryotischer Organismus ist.

25. Transformierter Wirt nach einem der Ansprüche 22 bis 24, worin der Wirt E. coli, ein anderer Darm-Organismus als E. coli, eine Pseudomonas- oder Bacillusart ist.

26. Transformierter Wirt nach Anspruch 25, worin der Wirt ein E. coli K12-Derivat ausgewählt aus JM109 und Y1090 ist.

27. Verfahren zur Transformation eines Wirts, um einen transformierten Wirt nach einem der Ansprüche 22 bis 26 zu liefern, wobei das Verfahren umfaßt, daß man einen Wirt bereitstellt, den Wirt kompetent für die Transformation macht und in den Wirt ein rekombinantes DNA-Molekül nach einem der Ansprüche 13 bis 18 einführt.

28. Expressionsprodukt eines transformierten Wirts nach einem der Ansprüche 22 bis 26, wobei das Produkt ein Antigen nach einem der Ansprüche 1 bis 6 umfaßt.

29. Expressionsprodukt nach Anspruch 28 in gereinigter Form.

30. Expressionsprodukt nach Anspruch 28 oder Anspruch 29, umfassend eine erste Polypeptidsequenz, die homolog mit dem Wirt ist und eine zweite Polypeptidsequenz, die eine Aminosäuresequenz ist, die ein Antigen nach einem der Ansprüche 1 bis 6 kodiert.

31. Expressionsprodukt nach Anspruch 30, worin die erste Polypeptidsequenz die gesamte β-Galactosidase oder das gesamte Tra-T oder ein Teil davon ist und der Wirt E. coli ist.

32. Verfahren zur Biosynthese eines proteinhaltigen Produktes umfassend ein Antigen nach einem der Ansprüche 1 bis 6, wobei das Verfahren umfaßt, daß man einen Wirt mit einem rekombinanten DNA-Molekül nach einem der Ansprüche 14 bis 18 so transformiert, daß der Wirt ein proteinhaltiges Produkt exprimieren kann, das ein Antigen nach einem der Ansprüche 1 bis 6 einschließt; den Wirt züchtet, um eine Expression zu erhalten und das proteinhaltige Produkt sammelt.

33. Impfstoff, umfassend eine wirksame Menge eines oder mehrerer Antigene nach einem der Ansprüche 1 bis 6, Expressionsprodukte nach einem der Ansprüche 28 bis 31 zusammen mit einem pharmazeutisch annehmbaren Träger, verdünnungsmittel, Hilfsstoff und/oder Adjuvans.

34. Impfstoff nach Anspruch 33, der für die orale Verabreichung geeignet ist oder in injizierbarer Form ist.

35. Antikörper, hergestellt als Ergebnis der Impfung eines Wirts durch Verabreichung von einem oder mehreren Antigenen nach einem der Ansprüche 1 bis 6, von Expressionsprodukten nach einem der Ansprüche 28 bis 31 und/oder von Impfstoffen nach einem der Ansprüche 33 und 34 an einen Wirt.

36. Verfahren zur Herstellung eines Antigens nach einem der Ansprüche 1 bis 6, wobei das Verfahren umfaßt, daß man die exkretorische/sekretorische Flüssigkeit einer parasitischen Stufe einer parasitischen Nematodenart sammelt; die Flüssigkeit durch Linsenlektin-Chromatographie mit Methylmannosid als Elutionsmittel fraktioniert; gebundene und ungebundene Fraktionen sammelt; mit SDS-Gel-Elektrophorese weiter fraktioniert und das Antigen elektroeluiert.

37. Verfahren zur Herstellung eines fusionierten Gens nach Anspruch 24, wobei das Verfahren umfaßt, daß man einen Promotor, ein Translations-Startsignal und eine DNA-Sequenz nach einem der Ansprüche 8 bis 11 bereitstellt und den Promotor, das Translations-Startsignal und die DNA-Sequenz operativ verbindet.

38. Verfahren zur Herstellung eines Impfstoffs nach einem der Ansprüche 33 und 34, wobei das Verfahren umfaßt, daß man eine wirksame Menge mindestens eines Antigens nach einem der Ansprüche 1 bis 6 und/oder Expressionsprodukte nach einem der Ansprüche 28 bis 31 mit einem pharmazeutisch annehmbaren Träger, verdünnungsmittel, Hilfsstoff und/oder Adjuvans vermischt.

39. Verfahren zur Herstellung eines Antikörpers nach Anspruch 35, wobei das Verfahren umfaßt:
daß man einen immunoreaktiven Wirt mit einer wirksamen Menge eines Antigens nach einem der Ansprüche 1 bis 6, einem Expressionsprodukt nach einem der Ansprüche 28 bis 31 oder einem Impfstoff nach einem der Ansprüche 33 und 34 immunisiert.

40. Antigen nach einem der Ansprüche 1 bis 6, Expressionsprodukt nach einem der Ansprüche 28 bis 31 und/oder Impfstoff nach einem der Ansprüche 33 oder 34 zur Verwendung für ein Verfahren zur Behandlung einer Infektion mit einem parasitischen Nematoden.

41. Antikörper nach Anspruch 35 zur Verwendung in einem Verfahren, um einen Wirt passiv gegen eine Infektion mit dem parasitischen Nematoden zu schützen.

## Revendications

1. Antigène comprenant une protéine d'excrétion/sécrétion, dérivée d'un stade parasitaire de Trichostrongylos colubriformis et capable de conférer à un hôte une immunité passive protectrice contre une infection par un nématode parasitaire, caractérisé en ce qu'il possède :
un poids moléculaire approximatif de 11kD, comme estimé par SDS-PAGE et comprend la séquence d'aminoacides qui suit : 
Met Leu Tyr Lys Lys Leu Arg Ser Gln Gly Asn Phe Arg Lys
Asn Asp Ser Ala Tyr Phe Lys Leu Glu Asn Lys Arg Glu Leu
Lys Gly Asp Asn Leu Pro Val Glu Glu Lys Val Arg Gln Thr
Ile Glu Lys Phe Lys Asp Asp Val Ser Glu Ile Arg Arg Leu
Ala Asp Asp Ser Asp Phe Gly Cys Asn Gly Lys Glu Thr Gly
Gly Ala Met His Ile Val Cys Phe Phe Gln Lys Asn Tyr Asp
Trp Met Lys Gly Gln Trp Gln Asn
un poids moléculaire approximatif de 17kD comme estimé par SDS-PAGE et comprend la séquence d'aminoacides suivant : 
Arg Phe Leu Leu Leu Ala Ala Phe Val Ala Tyr Ala Try Ala
Lys Ser Asp Glu Glu Ile Arg Lys Asp Ala Leu Ser Ala Leu
Asp Val Val Pro Leu Gly Ser Thr Pro Glu Lys Leu Glu Asn Gly
un poids moléculaire approximatif de 30kD comme estimé par SDS-PAGE et comprend la séquence d'aminoacides suivante : 
Ala Asn Asn Lys Gln Gln Thr Asp Ile Glu Gln Leu Met Pro
Lys Tyr Asn Ser Thr Phe Ala Lys Met Asn Gly Asn Tyr Ser
Tyr Lys Leu Ile Trp Asp Asp Ser Met Val Ser Asp Ala Leu
Gln Glu Ala Lys Glu Gln Tyr Ser Thr Asn Ala Thr Phe Lys
Ile Arg Arg Arg Lys Val Phe Ile Lys Gly Asp Asn Ala Thr
Met Glu Glu Lys Val Glu Gly Ala Leu Lys Tyr Pro Val Leu
Arg Ala Asp Lys Phe Leu Arg Arg Leu Leu Trp Phe Thr His
Tyr Ala Cys Asn Gly Tyr Tyr Asp Thr Lys Gly Gly His Asp
Val Leu Thr Val Ala Cys Leu Tyr Arg Glu Ile Asp Tyr Lys
Asn Ser His Tyr
un poids moléculaire approximatif de 37kD comme estimé par SDS-PAGE et comprend la séquence d'aminoacides suivante : Lys Ser Ile Leu Arg Leu et 
Lys Val Ile Pro Xaa Asn Pro Pro Ile Lys Asp Thr
Pro,
ou un poids moléculaire approximatif de 81kD comme mesuré par SDS-PAGE et comprend la séquence d'aminoacides aminoterminale suivante : Ser Xaa Ser Leu Lys Asp;
ou comprend une partie, un analogue, un homologue, ou des combinaisons de ceux-ci, de ladite protéine d'excrétion/sécrétion.

2. Antigène comprenant une protéine d'excrétion/sécrétion suivant la revendication 1, possédant un poids moléculaire approximatif de 11kD comme estimé par SDS-PAGE.

3. Antigène comprenant une protéine d'excrétion/sécrétion suivant la revendication 1, possédant un poids moléculaire approximatif de 17kD comme estimé par SDS-PAGE.

4. Antigène comprenant une protéine d'excrétion/sécrétion suivant la revendication 1, possédant un poids moléculaire approximatif de 30kD comme estimé par SDS-PAGE.

5. Antigène comprenant une protéine d'excrétion/sécrétion suivant la revendication 1, possédant un poids moléculaire approximatif de 37kD comme estimé par SDS-PAGE.

6. Antigène comprenant une protéine d'excrétion/sécrétion suivant la revendication 1, possédant un poids moléculaire approximatif de 81kD comme estimé par SDS-PAGE.

7. Séquence de nucléotides encodant la séquence d'aminoacides d'un antigène suivant l'une quelconque des revendications 1 à 6.

8. Séquence de nucléotides suivant la revendication 7, caractérisée en ce que la séquence de nucléotides est une séquence d'ADN.

9. Séquence d'ADN suivant la revendication 8, qui comprend :

GAA TTC GGG GGC AAC ACT TAC AGT GCA AAC AAT AAG CAA CAG ACC
GAC ATA GAA CAA CTC ATG CCC AAA TAT AAC TCG ACG TTC GCG AAG
ATG AAT GGA AAC TAT AGT TAT AAG CTG ATC TGG GAT GAC AGC ATG
GTA TCT GAT GCG CTG CAA GAA GCA AAG GAG CAA TAC AGT ACG AAT
GCT ACC TTC AAG ATC CGT CGG AGA AAG GTG TTC ATA AAG GGC GAT
AAC GCA ACG ATG GAG GAA AAA GTG GAG GGA GCT CTG AAG TAC CCC
GTC TTG AGA GCC GAT AAA TTT CTT CGC CGT CTT CTC TGG TTC ACA
CAC TAC GCA TGC AAT GGA TAT TAC GAT ACG AAA GGT GGA CAC GAT

GTC CTG ACT GTC GCG TGT CTC TAC AGA GAG ATC GAT TAC AAA AAT
TCT CAC TAT TAG AAA GCA GTC AAC AAA AAC AGC AGA GTA AAC TGA
CTG CAC ATT TCC GCA GTT TTT GAA TAA ATA CTT GAT GCA ACT CAA
AAA AAA AAA AAA

10. Séquence d'ADN suivant la revendication 8, qui comprend :

ACT ACA AGA CCC CAA TTG TAC ACG AAA TTC TTC AAC GAA GAA AAC
AGC CTA AAT CTG AGA TGG AAC CAC ATA TGT CGC AGC ATG CTC TAC
AAG AAA TTG AGA AGC CAG GGA AAT TTT CGC AAA AAT GAT TCA GCA
TAT TTC AAG CTC GAA AAC AAG AGG GAA CTG AAG GGA GAC AAT CTA
CCA GTG GAG GAG AAA GTA CGC CAA ACT ATT GAA AAA TTC AAG GAT
GAT GTA AGC GAA ATC AGA CGT CTT GCT GAT GAT TCG GAT TTT GGA
TGC AAC GGC AAA GAA ACC GGG GGT GCA ATG CAC ATT GTG TGT TTC
TTC CAG AAG AAT TAT GAC TGG ATG AAA GGA CAA TGG CAA AAC TGA
TTT TTC TGA AGT ACT TGT TGG ATT CTT CGT AGA ATC GAT GCA CAA
AAT ACC TTT TTT GGG AGA CAA CTT CGC ATA AAA CTT CTC GAT GAA
AAA AAA AAA AAA A

11. Séquence d'ADN suivant la revendication 8, qui comprend :

[?]GG TTC CTT CTT CTA GCA GCG TTC GTC GCC TAT GCG TAT GCA AAG
TCA GAT GAA GAA ATC CGA AAA GAT GCA CTA TCT GCT CTG GAT GTA
GTT CCA CTG GGT TCG ACT CCC GAA AAA CTG GAA AAT GGC

12. Procédé de sélection d'une séquence d'ADN ou d'ARN codant pour un antigène suivant l'une quelconque des revendications 1 à 6, caractérisé en ce que l'on prend une ou plusieurs séquences d'ADN ou d'ARN, on détermine laquelle des séquences s'hybride avec une séquence d'ADN ou d'ARN dont on sait qu'elle code pour un antigène suivant l'une quelconque des revendications 1 à 6, ou bien on prend un antisérum de l'antigène et on identifie des combinaisons hôte-vecteur qui expriment l'antigène.

13. Molécule d'ADN recombiné comprenant une séquence d'ADN suivant l'une quelconque des revendications 8 à 11 et l'ADN vecteur.

14. Molécule d'ADN recombiné suivant la revendication 13, comprenant en outre une séquence de commande de l'expression opérativement liée à la séquence d'ADN.

15. Molécule d'ADN recombiné suivant la revendication 14, caractérisée en ce que la séquence de commande de l'expression est choisie parmi le gène de β-galactosidase de E. coli, l'opéron tryptophane, le gène Tra-T de E. coli. Le promoteur gauche du bactériophage lambda, le promoteur tac, le promoteur Cup 1 et le promoteur précoce SV40.

16. Molécule d'ADN recombiné suivant l'une quelconque des revendications 13 à 15, caractérisée en ce que l'ADN vecteur est l'ADN plasmidique.

17. Molécule d'ADN recombiné suivant la revendication 13 ou la revendication 14, caractérisée en ce que l'ADN vecteur est l'ADN de bactériophage.

18. Molécule d'ADN recombiné suivant la revendication 17, caractérisée en ce que l'ADN de bactériophage est choisi parmi le bactériophage lambda et ses dérivés, y compris lambda gt10 et lambda gT11.

19. Gène fusionné comprenant un promoteur, un signal d'amorce ou de départ de traduction et une séquence d'ADN suivant l'une quelconque des revendications 8 à 11.

20. Procédé de préparation d'une molécule d'ADN recombiné suivant l'une quelconque des revendications 13 à 18, caractérisé en ce qu'il comprend la prise d'un insert d'ADN comprenant une séquence d'ADN suivant l'une quelconque des revendications 8 à 11 et l'introduction de l'insert d'ADN dans l'ADN vecteur.

21. Procédé suivant la revendication 20, caractérisé en ce que l'ADN inséré est introduit dans le vecteur de clonage dans l'espacement correct et le cadre de lecture correct par rapport à une séquence de commande de l'expression.

22. Hôte transformé, transformé avec au moins une molécule d'ADN recombiné suivant l'une quelconque des revendications 13 à 18.

23. Hôte transformé suivant la revendication 22, caractérisé en ce que l'hôte est capable d'exprimer un antigène suivant l'une quelconque des revendications 1 à 6.

24. Hôte transformé suivant la revendication 22 ou la revendication 23, caractérisé en ce que l'hôte est une cellule bactérienne, une levure, y compris la souche de Saccharomyces cerevisiae CL13-ABSY86 d'autres champignons, des cellules de vertébrés, des cellules d'insectes, des cellules de plantes, des cellules humaines, des cellules de tissu humain, des virus vivants, comme le virus de la vaccine ou le baculovirus, ou un organisme eukaryote entier.

25. Hôte transformé suivant l'une quelconque des revendications 22 à 24, caractérisé en ce que l'hôte est E. coli, un organisme entérique autre que E. coli, une espèce de Pseudomonas ou de Bacillus.

26. Hôte transformé suivant la revendication 25, caractérisé en ce que l'hôte est un dérivé d'E. coli K12 choisi parmi JH109 et Y1090.

27. Procédé pour transformer un hôte de manière à obtenir un hôte transformé suivant l'une quelconque des revendications 22 à 26, caractérisé en ce qu'il consiste à prendre un hôte, à rendre l'hôte compétent pour la transformation et à introduire dans l'hôte une molécule d'ADN recombiné suivant l'une quelconque des revendications 13 à 18.

28. Produit d'expression d'un hôte transformé suivant l'une quelconque des revendications 22 à 26, lequel produit comprend un antigène suivant l'une quelconque des revendications 1 à 6.

29. Produit d'expression suivant la revendication 28, sous forme purifiée.

30. Produit d'expression suivant la revendication 28 ou la revendication 29, qui comprend une première séquence de polypeptides homologue à l'hôte et une seconde séquence de polypeptides qui est une séquence d'aminoacides codant pour un antigène suivant l'une quelconque des revendications 1 à 6.

31. Produit d'expression suivant la revendication 30, caractérisé en ce que la première séquence de polypeptides est la totalité ou une partie de β-galactosidase ou de Tra-T et de l'hôte E. coli.

32. Procédé de biosynthèse d'un produit protéinique comprenant un antigène suivant l'une quelconque des revendications 1 à 6, caractérisé en ce que l'on transforme un hôte avec une molécule d'ADN recombiné suivant l'une quelconque des revendications 14 à 18, en sorte que l'hôte soit capable d'exprimer un produit protéinique qui inclut un antigène suivant l'une quelconque des revendications 1 à 6, on cultive l'hôte pour obtenir l'expression et on recueille le produit protéinique.

33. Vaccin comprenant une quantité efficace d'un ou plusieurs antigènes suivant l'une quelconque des revendications 1 à 6, des produits d'expression suivant l'une quelconque des revendications 28 à 31, en même temps qu'un adjuvant, excipient, diluant et/ou véhicule, pharmaceutiquement acceptable.

34. Vaccin suivant la revendication 32, convenant à l'administration par la voie orale, ou sous une forme injectable.

35. Anticorps préparé par suite de la vaccination d'un hôte par l'administration d'un ou plusieurs antigènes suivant l'une quelconque des revendications 1 à 6, produits d'expression suivant l'une quelconque des revendications 28 à 31 et/ou vaccins suivant l'une quelconque des revendications 33 et 34, à l'hôte

36. Procédé de préparation d'un antigène suivant l'une quelconque des revendications 1 à 6, caractérisé en ce que l'on recueille des liquides d'excrétion/sécrétion provenant d'un stade parasitaire d'une espèce de nématode parasitaire, on fractionne les liquides par chromatographie sur lectine de lentille avec du méthylmannoside comme éluant, on recueille les fractions liées et non liées, on fractionne davantage par électrophorèse sur gel-SDS et on électro-élue l'antigène.

37. Procédé de préparation d'un gène fusionné suivant la revendication 24, caractérisé en ce que l'on prend un promoteur, un signal d'amorce ou de départ de traduction et une séquence d'ADN suivant l'une quelconque des revendications 8 à 11 et on lie opérativement le promoteur, le signal d'amorce de traduction et la séquence d'ADN.

38. Procédé de préparation d'un vaccin suivant l'une quelconque des revendications 33 et 34, caractérisé en ce que l'on mélange une quantité efficace d'au moins un antigène suivant l'une quelconque des revendications 1 à 6 et/ou des produits d'expression suivant l'une quelconque des revendications 28 à 31, à un adjuvant, excipient, diluant et/ou un véhicule pharmaceutiquement acceptable.

39. Procédé de préparation d'un anticorps suivant la revendication 35, caractérisé en ce que
on immunise un hôte immunosensible avec une quantité efficace d'antigène suivant l'une quelconque des revendications 1 à 6, un produit d'expression suivant l'une quelconque des revendications 28 à 31, ou un vaccin suivant l'une quelconque des revendications 32 et 33.

40. Antigène suivant l'une quelconque des revendications 1 à 6, produit d'expression suivant l'une quelconque des revendications 28 à 31 et/ou vaccins suivant l'une quelconque des revendications 33 et 34, à utiliser pour la mise en oeuvre d'une méthode de traitement d'une infection due à un nématode parasitaire.

41. Anticorps suivant la revendication 35, à utiliser pour la mise en oeuvre d'une méthode de protection passive d'un hôte contre une infection par un nématode parasitaire.
